# EUROPEAN PATENT SPECIFICATION

(11) **EP 2 318 367 B1**
(45) Date of publication and mention of the grant of the patent: **20.03.2013**
(21) Application number: 09738535.5
(22) Date of filing: 29.04.2009
(51) Int. Cl.: C07D 211/56, C07D 401/12, C07D 417/14, C07D 471/04, C07D 493/04, A61K 31/4523, A61P 25/00

(54) **PIPERIDINE AND PYRROLIDINE COMPOUNDS**
PIPERIDIN- UND PYRROLIDINVERBINDUNGEN
COMPOSÉS DE PIPÉRIDINE ET DE PYRROLIDINE

(30) Priority: 30.04.2008 WO PCT/IB2008/051673
(43) Date of publication of application: 11.05.2011
(73) Proprietor: Actelion Pharmaceuticals Ltd., 4123 Allschwil (CH)
(72) Inventor: AISSAOUI, Hamed, F-68840 Pulversheim (FR); BOSS, Christoph, CH-4123 Allschwil (CH); KOBERSTEIN, Ralf, 79539 Lörrach (DE); SIEGRIST, Romain, CH-4123 Allschwil (CH); SIFFERLEN, Thierry, F-68220 Wentzwiller (FR); TRACHSEL, Daniel, CH-4416 Bubendorf (CH)
(74) Representative: Velker, Jörg
(86) International application number: PCT/IB2009/051735
(87) International publication number: WO 2009/133522

(56) References cited:
- WO-A-01/96302

## Description

The present invention relates to novel piperidine and pyrrolidine compounds of formula (I) and their use as pharmaceuticals. The invention also concerns related aspects including processes for the preparation of the compounds, pharmaceutical compositions containing one or more compounds of formula (I), and especially their use as orexin receptor antagonists.

Orexins (orexin A or OX-A and orexin B or OX-B) are novel neuropeptides found in 1998 by two research groups, orexin A is a 33 amino acid peptide and orexin B is a 28 amino acid peptide (Sakurai T. et al., Cell, 1998, 92, 573-585). Orexins are produced in discrete neurons of the lateral hypothalamus and bind to the G-protein-coupled receptors (OX₁ and OX₂ receptors). The orexin-1 receptor (OX₁) is selective for OX-A, and the orexin-2 receptor (OX₂) is capable to bind OX-A as well as OX-B. Orexins are found to stimulate food consumption in rats suggesting a physiological role for these peptides as mediators in the central feedback mechanism that regulates feeding behaviour (Sakurai T. et al., Cell, 1998, 92, 573-585). On the other hand, it was also observed that orexins regulate states of sleep and wakefulness opening potentially novel therapeutic approaches to narcolepsy as well as insomnia and other sleep disorders (Chemelli R.M. et al., Cell, 1999, 98, 437-451).

Orexin receptors are found in the mammalian brain and may have numerous implications in pathologies such as dysthymic, mood, psychotic and anxiety disorders; diabetes and appetite, taste, eating, or drinking disorders; hypothalamic diseases; disturbed biological and circadian rhythms; sleep disturbances associated with diseases such as neurological disorders, neuropathic pain and restless leg syndrome; insomnias related to psychiatric disorders; sleep apnea; narcolepsy; idiopathic insomnias; parasomnias; benign prostatic hypertrophy; all dementias and cognitive dysfunctions in the healthy population and in psychiatric and neurologic disorders; and other diseases related to general orexin system dysfunctions.

The present invention provides piperidine and pyrrolidine derivatives, which are non-peptide antagonists of human orexin receptors. These compounds are in particular of potential use in the treatment of e.g. eating disorders, drinking disorders, sleep disorders, or cognitive dysfunctions in psychiatric and neurologic disorders.

Up to now, several low molecular weight compounds are known having a potential to antagonise either specifically OX₁ or OX₂, or both receptors at the same time. Piperidine derivatives useful as orexin receptor antagonists are disclosed in WO01/096302.

Nitrogenous heterocyclic compounds useful for a disease for which sodium channel inhibition is effective are described in EP 1484327. 1,3-Substituted cycloamino derivatives useful as histamine-3 receptor antagonists are described in WO 2006/011042.
i) The present invention consists of compounds of formula (I) 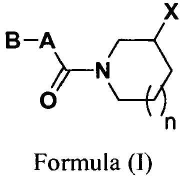 wherein
   A represents a phenyl-group, wherein the phenyl is unsubstituted, or mono-substituted with (C₁₋₄)alkyl; or A represents 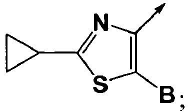 B represents phenyl, which is unsubstituted, or mono-, di-, or tri-substituted, wherein the substituents are independently selected from the group consisting of (C₁₋₄)alkyl, (C₁₋₄)alkoxy, trifluoromethyl, cyano and halogen;
   n represents the integer 0 or 1;
   X represents -NH-R¹ or -NH-C(O)-R²;
   R¹ represents heteroaryl, wherein said heteroaryl is selected from the group consisting of pyrimidinyl, which is mono-, or di-substituted, wherein the substituents are independently selected from (C₁₋₄)alkoxy, trifluoromethyl, (C₁₋₄)alkyl-thio-, and (C₁₋₄)alkoxy-carbonyl-, or which is di-substituted, wherein one substituent is methyl and the other substituent is selected from (C₁₋₄)alkoxy, trifluoromethyl, (C₁₋₄)alkyl-thio-, and (C₁₋₄)alkoxy-carbonyl-; pyridinyl which is unsubstituted, mono-, or di-substituted, wherein the substituents are independently selected from (C₁₋₄)alkyl, (C₁₋₄)alkoxy, trifluoromethyl, halogen, (C₁₋₄)alkyl-carbonyl-, and nitro; benzothiazolyl; benzimidazolyl; and quinoxalinyl which is unsubstituted, or di-substituted, wherein both substituents are independently halogen;
   R² represents phenyl, which is di-substituted (notably in position 2 and 3), wherein the substituents are independently selected from (C₁₋₄)alkyl and halogen; or
   R² represents heteroaryl, wherein said heteroaryl is selected from the group consisting of pyrazolyl, indolyl, indazolyl, benzisoxazolyl, quinolinyl, isoquinolinyl, imidazo[1,2-a]pyridyl, 1H-pyrrolopyridyl (notably 1H-pyrrolo[3,2-b]pyridyl and 1H-pyrrolo[2,3-b]pyridyl), and 2,3-dihydro-thieno[3,4-b][1,4]dioxinyl; wherein said groups are independently unsubstituted, mono-, or di-substituted, wherein the substituents are independently selected from (C₁₋₄)alkyl; or
   R² represents heterocyclyl, wherein said heterocyclyl is selected from the group consisting of 2,3-dihydro-benzo[1,4]dioxinyl, 2,3-dihydro-benzofuranyl, benzo[1,3]dioxolyl, 4H-benzo[1,3]dioxinyl, 2H-chromenyl, and chromanyl.

The compounds of formula (I) may contain one or more stereogenic or asymmetric centers, such as one or more asymmetric carbon atoms. The compounds of formula (I) may thus be present as mixtures of stereoisomers or preferably as pure stereoisomers. Mixtures of stereoisomers may be separated in a manner known to a person skilled in the art.

In this patent application, an arrow shows the point of attachment of the radical drawn. For example, the radical drawn below 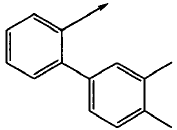 is the 3',4'-dimethyl-biphenyl-2-yl group.

In case "A" represents "phenyl-group, wherein the phenyl is unsubstituted, or mono-substituted with (C₁₋₄)alkyl", said phenyl-group is preferably unsubstituted. In addition to the above-mentioned substituents, the group "A" is also substituted by the substituent "B", wherein B is preferably attached in ortho position to the point of attachment of the carbonyl group which links A to the rest of the molecule.

Preferably a phenyl group as used for the substituent "B" is unsubstituted, or mono-, or di-substituted, wherein the substituents are independently selected from the group consisting of (C₁₋₄)alkyl, (C₁₋₄)alkoxy, trifluoromethyl and halogen. In a further preferred embodiment, the substituents are independently selected from the group consisting of methyl, methoxy, trifluoromethyl, fluorine and chlorine. Examples are phenyl, 2-methyl-phenyl, 3-methyl-phenyl, 4-methyl-phenyl, 3,4-dimethylphenyl, 3-methoxyphenyl, 4-methoxyphenyl, 3,4-dimethoxyphenyl, 4-fluorophenyl, 3-fluorophenyl, 2-fluorophenyl, 3-fluoro-4-methylphenyl, 3-trifluoromethylphenyl, 3-chlorophenyl, 3-bromophenyl, 3-fluoro-5-trifluoromethylphenyl, and 4-cyanophenyl. Especially, examples are 3-methyl-phenyl, 3,4-dimethylphenyl, and 4-fluorophenyl.

In another embodiment, in case "A" and "B" both represent "phenyl" the combination "A-B" preferably means a biphenyl group (especially a biphen-2-yl group) which is unsubstituted for "A" and unsubstituted, or mono-, di- or tri-substituted (preferably mono-, or di-substituted) for "B", wherein the substituents are independently selected from the group consisting of (C₁₋₄)alkyl, (C₁₋₄)alkoxy, trifluoromethyl and halogen, especially from (C₁₋₄)alkyl, (C₁₋₄)alkoxy and halogen. Further preferred examples of substituents for "B" are methyl and methoxy (notably methyl).

Examples of such biphenyl groups "A-B" are:

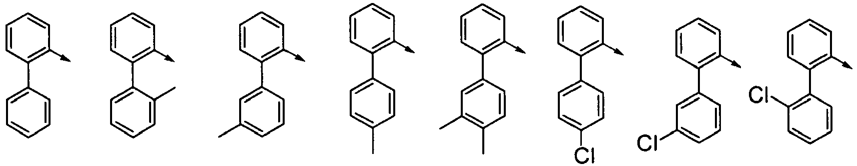

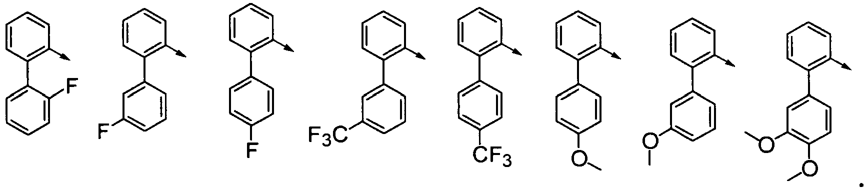

Preferred examples of such biphenyl groups "A-B" are:

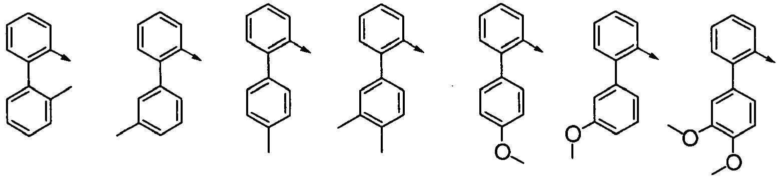

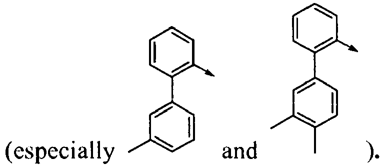

The 2-cyclopropyl-thiazolyl-group as defined for group "A", is also substituted by the substituent "B", whereby B is attached in ortho position to the point of attachment of the carbonyl group which links A to the rest of the molecule.

Examples of groups "A-B" wherein "A" represents a 2-cyclopropyl-thiazolyl-group are:

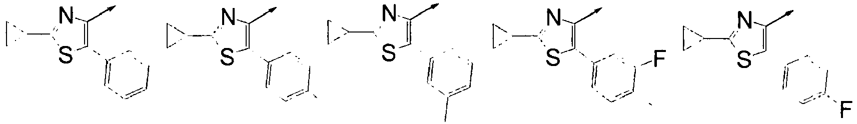

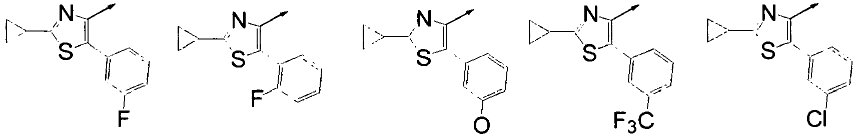

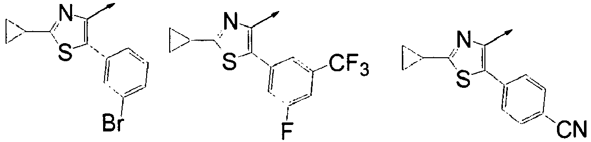

Especially, examples of such groups are:

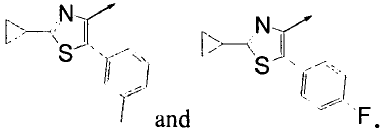

Examples wherein "R¹" represents "heteroaryl" are pyrimidin-2-yl, which is mono-, or di-substituted, wherein the substituents are independently selected from (C₁₋₄)alkoxy, trifluoromethyl, (C₁₋₄)alkyl-thio-, and (C₁₋₄)alkoxy-carbonyl- (notably from (C₁₋₄)alkoxy and trifluoromethyl), or which is di-substituted, wherein one substituent is methyl and the other substituent is selected from (C₁₋₄)alkoxy, trifluoromethyl, (C₁₋₄)alkyl-thio-, and (C₁₋₄)alkoxy-carbonyl- (notably (C₁₋₄)alkoxycarbonyl-); pyridin-2-yl and pyridin-3-yl which groups are unsubstituted, mono-, or di-substituted, wherein the substituents are independently selected from (C₁₋₄)alkyl, (C₁₋₄)alkoxy, trifluoromethyl, halogen, (C₁₋₄)alkyl-carbonyl-, and nitro (notably from trifluoromethyl and nitro); benzothiazol-2-yl; benzimidazol-2-yl; and quinoxalin-2-yl which is unsubstituted, or di-substituted, wherein both substituents are independently halogen (notably both are fluorine).

Particular examples of R¹ representing "heteroaryl" are selected from:

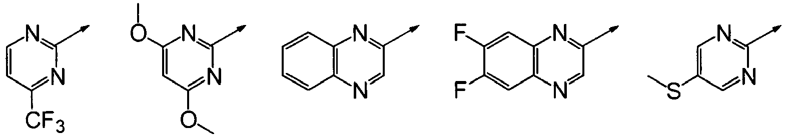

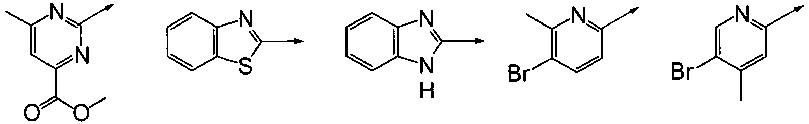

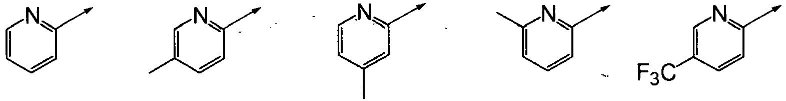

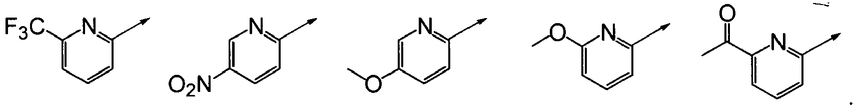

Especially, such examples of R¹ representing "heteroaryl" are selected from:

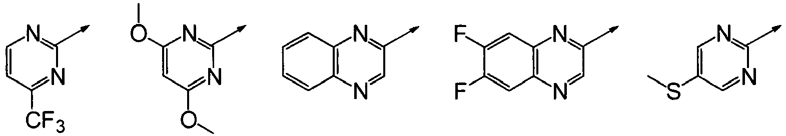

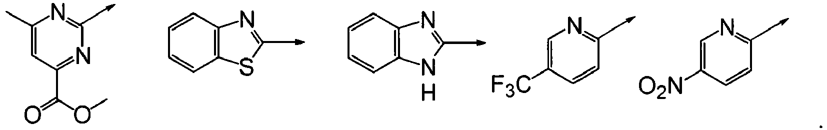

Examples wherein "R²" represents "heteroaryl" are pyrazol-3-yl, indol-3-yl, indazol-3-yl, benzisoxazol-3-yl, quinolin-8-yl, isoquinolin-1-yl, imidazo[1,2-a]pyridine-3-yl, 1H-pyrrolo[3,2-b]pyridin-4-yl, 1H-pyrrolo[2,3-b]pyridin-4-yl, 1H-pyrrolo[2,3-b] pyridin-5-yl, and 2,3-dihydro-thieno[3,4-b][1,4]dioxin-5-yl; wherein the above-mentioned groups are independently unsubstituted, mono-, or di-substituted, wherein the substituents are independently selected from (C₁₋₄)alkyl.

In particular, the above mentioned "heteroaryl" groups as used for the substituent "R²" are preferably substituted as follows: pyrazolyl groups are di-substituted, wherein both substituents are independently selected from (C₁₋₄)alkyl; indolyl and indazolyl groups are mono-substituted (notably on a nitrogen atom) with (C₁₋₄)alkyl (especially methyl); benzisoxazolyl, quinolinyl, isoquinolinyl, imidazo[1,2-a]pyridyl, pyrrolopyridyl, and 2,3-dihydro-thieno[3,4-b][1,4]dioxinyl groups are unsubstituted.

Particular examples of R² representing "heteroaryl" are selected from:

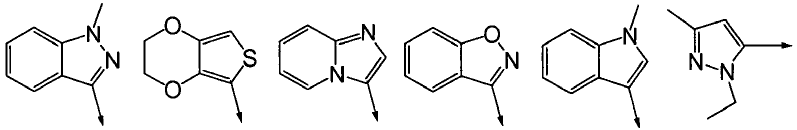

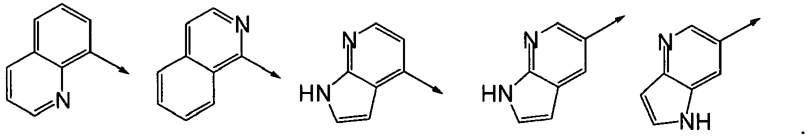

Examples wherein R² represents "phenyl, which is di-substituted, wherein the substituents are independently selected from (C₁₋₄)alkyl and halogen" are 3-chloro-2-methyl-phenyl, 3-fluoro-2-methylphenyl, and 2,3-dimethylphenyl.

The term "heterocyclyl", alone or in combination, means a phenyl ring fused to a saturated or partially unsaturated 5- to 6-membered ring containing 1 or 2 heteroatoms independently selected from oxygen and nitrogen; wherein the phenyl ring carries the point of attachment to the rest of the molecule. Examples of "heterocyclyl" as used for the substituent "R²" are 2,3-dihydro-benzo[1,4]dioxinyl, 2,3-dihydro-benzofuranyl, benzo[1,3]dioxolyl, 4*H*-benzo[1,3]dioxinyl, 2*H*-chromenyl, and chromanyl. A preferred example is 2,3-dihydro-benzo[1,4]dioxinyl, notably 2,3-dihydro-benzo[1,4]dioxin-5-yl.

The term "halogen" means fluorine, chlorine, or bromine, preferably fluorine or chlorine.

The term "(C₁₋₄)alkyl", alone or in combination, means a straight-chain or branched-chain alkyl group with 1 to 4 carbon atoms. Examples of (C₁₋₄)alkyl groups are methyl, ethyl, propyl, isopropyl, n-butyl, isobutyl, sec.-butyl or tert.-butyl. Preferred are methyl and ethyl. Most preferred is methyl.

The term "(C₁₋₄)alkoxy", alone or in combination, means a group of the formula (C₁₋₄)alkyl-O- in which the term "(C₁₋₄)alkyl" has the previously given significance, such as methoxy, ethoxy, n-propoxy, isopropoxy, n-butoxy, isobutoxy, sec.-butoxy or tert.-butoxy. Preferred are methoxy and ethoxy. Most preferred is methoxy.
ii) A further embodiment of the invention relates to compounds of formula (I) according to embodiment i) which are also compounds of formula (Ia), wherein the stereogenic center in position 3 of the piperidine or pyrrolidine ring is in absolute (R)-configuration: 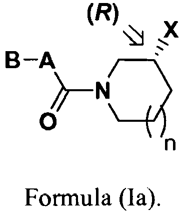
iii) A further embodiment of the invention relates to compounds of formula (I) according to embodiments i) or ii), wherein A represents a phenyl-group, wherein the phenyl is unsubstituted (preferred), or mono-substituted with (C₁₋₄)alkyl.
iv) A further embodiment of the invention relates to compounds of formula (I) according to embodiments i) or ii), wherein A represents 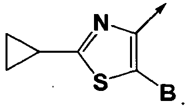
v) A further embodiment of the invention relates to compounds of formula (I) according to any one of embodiments i) to iv), wherein B represents phenyl, which is unsubstituted, or mono-, or di-substituted, wherein the substituents are independently selected from the group consisting of (C₁₋₄)alkyl, (C₁₋₄)alkoxy, trifluoromethyl and halogen.
vi) A further embodiment of the invention relates to compounds of formula (I) according to embodiment iii), wherein B represents phenyl, which is mono-, or di-substituted, wherein the substituents are independently selected from the group consisting of methyl and methoxy (notably methyl).
vii) A further embodiment of the invention relates to compounds of formula (I) according to embodiment iv), wherein B represents phenyl, which is unsubstituted, or (preferred) mono-, or di-substituted, wherein the substituents are independently selected from the group consisting of methyl, methoxy, trifluoromethyl, fluorine and chlorine (notably methyl and fluorine).
viii) A further embodiment of the invention relates to compounds of formula (I) according to any one of embodiments i) to vii), wherein n represents the integer 0.
ix) A further embodiment of the invention relates to compounds of formula (I) according to any one of embodiments i) to vii), wherein n represents the integer 1.
x) A further embodiment of the invention relates to compounds of formula (I) according to any one of embodiments i) to ix), wherein X represents -NH-R¹.
xi) A further embodiment of the invention relates to compounds of formula (I) according to any one of embodiments i) to ix), wherein X represents -NH-C(O)-R².
xii) A further embodiment of the invention relates to compounds of formula (I) according to embodiment x), wherein R¹ is selected from the group consisting of: 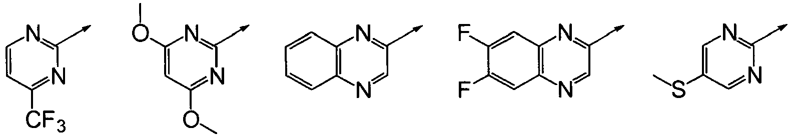 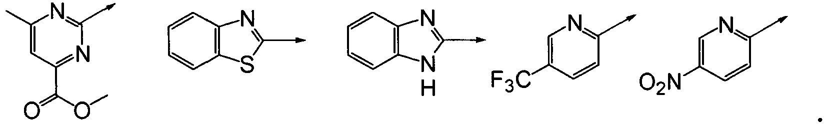
xiii) A further embodiment of the invention relates to compounds of formula (I) according to embodiment x), wherein R¹ is selected from the group consisting of: 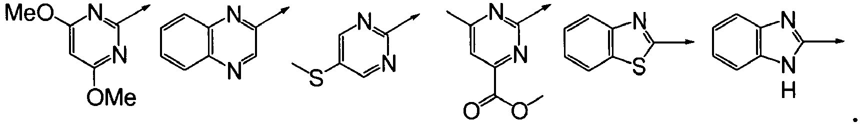
xiv) A further embodiment of the invention relates to compounds of formula (I) according to embodiment xi), wherein
   R² represents phenyl, which is di-substituted (notably in position 2 and 3), wherein the substituents are independently selected from (C₁₋₄)alkyl and halogen.
xv) A further embodiment of the invention relates to compounds of formula (I) according to embodiment xi), wherein
   R₂ represents heteroaryl, wherein said heteroaryl is selected from the group consisting of pyrazolyl, indolyl, indazolyl, benzisoxazolyl, quinolinyl, isoquinolinyl, imidazo[1,2-a]pyridyl, 1H-pyrrolopyridyl (notably 1H-pyrrolo[3,2-b]pyridyl and 1H-pyrrolo[2,3-b]pyridyl), and 2,3-dihydro-thieno[3,4-b][1,4]dioxinyl; wherein said groups are independently unsubstituted, mono-, or di-substituted, wherein the substituents are independently selected from (C₁₋₄)alkyl; or
   R² represents heterocyclyl, wherein said heterocyclyl is selected from the group consisting of 2,3-dihydro-benzo[1,4]dioxinyl, 2,3-dihydro-benzofuranyl, benzo[1,3]dioxolyl, 4*H*-benzo[1,3]dioxinyl, 2*H*-chromenyl, and chromanyl (notably 2,3-dihydro-benzo[1,4]dioxinyl).
xvi) A further embodiment of the invention relates to compounds of formula (I) according to embodiment xi) or xv), wherein
   R² represents heterocyclyl, wherein said heterocyclyl is selected from the group consisting of 2,3-dihydro-benzo[1,4]dioxinyl, 2,3-dihydro-benzofuranyl, benzo[1,3]dioxolyl, 4*H*-benzo[1,3]dioxinyl, 2*H*-chromenyl, and chromanyl (notably 2,3-dihydro-benzo[1,4]dioxinyl).
xvii) A further embodiment of the invention relates to compounds of formula (I) according to embodiment xi) or xv), wherein
   R² represents heteroaryl, wherein said heteroaryl is selected from the group consisting of pyrazolyl, indolyl, indazolyl, benzisoxazolyl, quinolinyl, isoquinolinyl, imidazo[1,2-a]pyridyl, 1H-pyrrolopyridyl (notably 1H-pyrrolo[3,2-b]pyridyl and 1H-pyrrolo[2,3-b]pyridyl), and 2,3-dihydro-thieno[3,4-b][1,4]dioxinyl; wherein said groups are independently unsubstituted, mono-, or di-substituted, wherein the substituents are independently selected from (C₁₋₄)alkyl.
xviii) A further embodiment of the invention relates to compounds of formula (I) according to embodiment xi) in combination with embodiment iv), wherein R² is selected from the group consisting of: 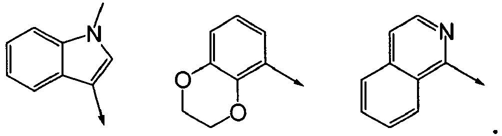
xix) A further embodiment of the invention relates to compounds of formula (I) according to embodiment xi) in combination with embodiment iii), wherein R² is selected from the group consisting of: 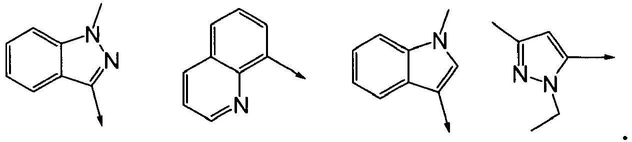
xx) A further embodiment of the invention relates to compounds of formula (I) according to embodiment i), which are selected from the group consisting of:
   (R)-(3',4'-Dimethyl-biphenyl-2-yl)-[3-(4-trifluoromethyl-pyrimidin-2-ylamino)-piperidin-1-yl]-methanone;
   (R)-[3-(4,6-Dimethoxy-pyrimidin-2-ylamino)-piperidin-1-yl]-(3',4'-dimethylbiphenyl-2-yl)-methanone;
   (R)-(3'-Methyl-biphenyl-2-yl)-[3-(4-trifluoromethyl-pyrimidin-2-ylamino)-piperidin-1-yl]-methanone;
   (R)-[3-(4,6-Dimethoxy-pyrimidin-2-ylamino)-piperidin-1-yl]-(3'-methyl-biphenyl-2-yl)-methanone;
   (R)-[3-(6,7-Difluoro-quinoxalin-2-ylamino)-piperidin-1-yl]-(3',4'-dimethyl-biphenyl-2-yl)-methanone;
   (R)-(3',4'-Dimethyl-biphenyl-2-yl)-[3-(quinoxalin-2-ylamino)-piperidin-1-yl]-methanone;
   (R)-(3',4'-Dimethyl-biphenyl-2-yl)-[3-(5-methylsulfanyl-pyrimidin-2-ylamino)-piperidin-1-yl]-methanone;
   (R)-2-[1-(3',4'-Dimethyl-biphenyl-2-carbonyl)-piperidin-3-ylamino]-6-methylpyrimidine-4-carboxylic acid methyl ester;
   (R)-[3-(Benzothiazol-2-ylamino)-piperidin-1-yl]-(3',4'-dimethyl-biphenyl-2-yl)-methanone;
   (R)-[3-(1H-Benzoimidazol-2-ylamino)-piperidin-1-yl]-(3',4'-dimethyl-biphenyl-2-yl)-methanone;
   (R)-[2-Cyclopropyl-5-m-tolyl-thiazol-4-yl]-[3-(4-trifluoromethyl-pyrimidin-2-ylamino)-piperidin-1-yl]-methanone;
   (R)-(2-Cyclopropyl-5-m-tolyl-thiazol-4-yl)-[3-(4,6-dimethoxy-pyrimidin-2-ylamino)-piperidin-1-yl]-methanone;
   (R)-[2-Cyclopropyl-5-(4-fluoro-phenyl)-thiazol-4-yl]-[3-(4-trifluoromethyl-pyrimidin-2-ylamino)-piperidin-1-yl]-methanone;
   (R)-[2-Cyclopropyl-5-(4-fluoro-phenyl)-thiazol-4-yl]-[3-(4,6-dimethoxy-pyrimidin-2-ylamino)-piperidin-1-yl]-methanone;
   Synthesis of (R)-1-Methyl-1H-indazole-3-carboxylic acid [1-(2-cyclopropyl-5-m-tolyl-thiazole-4-carbonyl)-piperidin-3-yl]-amide;
   (R)-2,3-Dihydro-thieno[3,4-b][1,4]dioxine-5-carboxylic acid [1-(2-cyclopropyl-5.-m-tolyl-thiazole-4-carbonyl)-piperidin-3-yl]-amide;
   (R)-Imidazo[1,2-a]pyridine-3-carboxylic acid [1-(2-cyclopropyl-5-m-tolyl-thiazole-4-carbonyl)-piperidin-3-yl]-amide;
   (R)-Benzo[d]isoxazole-3-carboxylic acid [1-(2-cyclopropyl-5-m-tolyl-thiazole-4-carbonyl)-piperidin-3-yl]-amide;
   (R)-3-Chloro-N-[1-(2-cyclopropyl-5-m-tolyl-thiazole-4-carbonyl)-piperidin-3-yl]-2-methyl-benzamide;
   (R)-N-[1-(2-Cyclopropyl-5-m-tolyl-thiazole-4-carbonyl)-piperidin-3-yl]-3-fluoro-2-methyl-benzamide;
   (R)-N-[1-(2-Cyclopropyl-5-m-tolyl-thiazole-4-carbonyl)-piperidin-3-yl]-2,3-dimethyl-benzamide;
   (R)-2,3-Dihydro-benzo[1,4]dioxine-5-carboxylic acid [1-(2-cyclopropyl-5-m-tolyl-thiazole-4-carbonyl)-piperidin-3-yl]-amide;
   (R)-1-Methyl-1H-indole-3-carboxylic acid [1-(2-cyclopropyl-5-m-tolyl-thiazole-4-carbonyl)-piperidin-3-yl]-amide;
   (R)-2-Ethyl-5-methyl-2H-pyrazole-3-carboxylic acid [1-(2-cyclopropyl-5-m-tolyl-thiazole-4-carbonyl)-piperidin-3-yl]-amide;
   (R)-Quinoline-8-carboxylic acid [-(2-cyclopropyl-5-m-tolyl-thiazole-4-carbonyl)-piperidin-3-yl]-amide;
   (R)-Isoquinoline-1-carboxylic acid [1-(2-cyclopropyl-5-m-tolyl-thiazole-4-carbonyl)-piperidin-3-yl]-amide;
   (R)-2,5-Dimethyl-2H-pyrazole-3-carboxylic acid [1-(2-cyclopropyl-5-m-tolyl-thiazole-4-carbonyl)-piperidin-3-yl]-amide;
   (R)-2,3-Dihydro-thieno[3,4-b][1,4]dioxine-5-carboxylic acid {1-[2-cyclopropyl-5-(4-fluoro-phenyl)-thiazole-4-carbonyl]-piperidin-3-yl}-amide;
   (R)-Imidazo[1,2-a]pyridine-3-carboxylic acid {1-[2-cyclopropyl-5-(4-fluoro-phenyl)-thiazole-4-carbonyl]-piperidin-3-yl}-amide;
   (R)-Benzo[d]isoxazole-3-carboxylic acid {1-[2-cyclopropyl-5-(4-fluoro-phenyl)-thiazole-4-carbonyl]-piperidin-3-yl}-amide;
   (R)-2,3-Dihydro-benzo[1,4]dioxine-5-carboxylic acid {1-[2-cyclopropyl-5-(4-fluorophenyl)-thiazole-4-carbonyl]-piperidin-3-yl}-amide;
   (R)-1-Methyl-1H-indole-3-carboxylic acid {1-[2-cyclopropyl-5-(4-fluoro-phenyl)-thiazole-4-carbonyl]-piperidin-3-yl}-amide;
   (R)-lsoquinoline-1-carboxylic acid {1-[2-cyclopropyl-5-(4-fluoro-phenyl)-thiazole-4-carbonyl]-piperidin-3-yl}-amide;
   (R)-1-Methyl-1H-indazole-3-carboxylic acid [1-(3'-methyl-biphenyl-2-carbonyl)-piperidin-3-yl]-amide;
   (R)-1-Methyl-1H-indole-3-carboxylic acid [1-(3'-methyl-biphenyl-2-carbonyl)-piperidin-3-yl]-amide;
   (R)-1H-Pyrrolo[2,3-b]pyridine-4-carboxylic acid [1-(3'-methyl-biphenyl-2-carbonyl)-piperidin-3-yl]-amide;
   (R)-2-Ethyl-5-methyl-2H-pyrazole-3-carboxylic acid [1-(3'-methyl-biphenyl-2-carbonyl)-piperidin-3-yl]-amide;
   (R)-Quinoline-8-carboxylic acid [1-(3'-methyl-biphenyl-2-carbonyl)-piperidin-3-yl]-amide;
   (R)-1H-Pyrrolo[2,3-b]pyridine-5-carboxylic acid [1-(3'-methyl-biphenyl-2-carbonyl)-piperidin-3-yl]-amide;
   (R)-1-Methyl-1H-indazole-3-carboxylic acid [1-(3',4'-dimethyl-biphenyl-2-carbonyl)-piperidin-3-yl]-amide;
   (R)-1-Methyl-1H-indole-3-carboxylic acid [1-(3',4'-dimethyl-biphenyl-2-carbonyl)-piperidin-3-yl]-amide;
   (R)-1H-Pyrrolo[2,3-b]pyridine-4-carboxylic acid [1-(3',4'-dimethyl-biphenyl-2-carbonyl)-piperidin-3-yl]-amide;
   (R)-2-Ethyl-5-methyl-2H-pyrazole-3-carboxylic acid [1-(3',4'-dimethyl-biphenyl-2-carbonyl)-piperidin-3-yl]-amide;
   (R)-Quinoline-8-carboxylic acid [1-(3',4'-dimethyl-biphenyl-2-carbonyl)-piperidin-3-yl]-amide;
   (R)-1H-Pyrrolo[2,3-b]pyridine-5-carboxylic acid [1-(3',4'-dimethyl-biphenyl-2-carbonyl)-piperidin-3-yl]-amide;
   (R)-1H-Pyrrolo[3,2-b]pyridine-6-carboxylic acid [1-(3',4'-dimethyl-biphenyl-2-carbonyl)-piperidin-3-yl]-amide;
   (R)-(3',4'-Dimethyl-biphenyl-2-yl)-[3-(5-trifluoromethyl-pyridin-2-ylamino)-piperidin-1-yl]-methanone;
   (R)-(3',4'-Dimethyl-biphenyl-2-yl)-[3-(5-nitro-pyridin-2-ylamino)-piperidin-1-yl]-methanone;
   (R)-(3'-Methyl-biphenyl-2-yl)-[3-(5-methylsulfanyl-pyrimidin-2-ylamino)-pyrrolidin-1-yl]-methanone;
   (R)-[3-(4,6-Dimethoxy-pyrimidin-2-ylamino)-pyrrolidin-1-yl]-(3'-methyl-biphenyl-2-yl)-methanone;
   (R)-(3'-Methyl-biphenyl-2-yl)-[3-(quinoxalin-2-ylamino)-pyrrolidin-1-yl]-methanone; (R)-(3'-Methyl-biphenyl-2-yl)-[3-(4-trifluoromethyl-pyrimidin-2-ylamino)-pyrrolidin-1-yl]-methanone;
   (R)-[3-(Benzothiazol-2-ylamino)-pyrrolidin-1-yl]-(3'-methyl-biphenyl-2-yl)-methanone;
   (R)-[3-(1H-Benzoimidazol-2-ylamino)-pyrrolidin-1-yl]-(3'-methyl-biphenyl-2-yl)-methanone;
   (R)-1-Methyl-1H-indole-3-carboxylic acid [1-(3'-methyl-biphenyl-2-carbonyl)-pyrrolidin-3-yl]-amide; and
   (R)-1-Methyl-1H-indazole-3-carboxylic acid [1-(3'-methyl-biphenyl-2-carbonyl)-pyrrolidin-3-yl]-amide.

Also part of the invention are compounds of formula (I) and (Ia) and salts, especially pharmaceutically acceptable salts thereof.

The term "pharmaceutically acceptable salts" refers to non-toxic, inorganic or organic acid and/or base addition salts. Reference can be made to "Salt selection for basic drugs", Int. J. Pharm. (1986), 33, 201-217.

Where the plural form is used for compounds, salts, pharmaceutical compositions, diseases or the like, this is intended to mean also a single compound, salt, disease or the like.

The compounds of formula (I) and (Ia) and their pharmaceutically acceptable salts can be used as medicaments, e.g. in the form of pharmaceutical compositions for enteral or parenteral administration.

A further aspect of the invention is a pharmaceutical composition containing at least one compound according to formula (I), or a pharmaceutically acceptable salt thereof, and a pharmaceutically acceptable carrier material.

The production of the pharmaceutical compositions can be effected in a manner which will be familiar to any person skilled in the art (see for example Remington, The Science and Practice of Pharmacy, 21 st Edition (2005), Part 5, "pharmaceutical Manufacturing" [published by Lippincott Williams & Wilkins]) by bringing the described compounds of formula (I) or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

The compounds of formula (I) and/or (Ia) may be used for the preparation of a medicament for the prevention or treatment of diseases selected from the group consisting of dysthymic disorders including major depression and cyclothymia, affective neurosis, all types of manic depressive disorders, delirium, psychotic disorders, schizophrenia, catatonic schizophrenia, delusional paranoia, adjustment disorders and all clusters of personality disorders; schizoaffective disorders; anxiety disorders including generalized anxiety, obsessive compulsive disorder, posttraumatic stress disorder, panic attacks, all types of phobic anxiety and avoidance; separation anxiety; all psychoactive substance use, abuse, seeking and reinstatement; all types of psychological or physical addictions, dissociative disorders including multiple personality syndromes and psychogenic amnesias; sexual and reproductive dysfunction; psychosexual dysfunction and addiction; tolerance to narcotics or withdrawal from narcotics; increased anaesthetic risk, anaesthetic responsiveness; hypothalamic-adrenal dysfunctions; disturbed biological and circadian rhythms; sleep disturbances associated with diseases such as neurological disorders including neuropathic pain and restless leg syndrome; sleep apnea; narcolepsy; chronic fatigue syndrome; insomnias related to psychiatric disorders; all types of idiopathic insomnias and parasomnias; sleep-wake schedule disorders including jet-lag; all dementias and cognitive dysfunctions in the healthy population and in psychiatric and neurological disorders; mental dysfunctions of aging; all types of amnesia; severe mental retardation; dyskinesias and muscular diseases; muscle spasticity, tremors, movement disorders; spontaneous and medication-induced dyskinesias; neurodegenerative disorders including Huntington's, Creutzfeld-Jacob's, Alzheimer's diseases and Tourette syndrome; Amyotrophic lateral sclerosis; Parkinson's disease; Cushing's syndrome; traumatic lesions; spinal cord trauma; head trauma; perinatal hypoxia; hearing loss; tinnitus; demyelinating diseases; spinal and cranial nerve diseases; ocular damage; retinopathy; epilepsy; seizure disorders; absence seizures, complex partial and generalized seizures; Lennox-Gastaut syndrome; migraine and headache; pain disorders; anaesthesia and analgesia; enhanced or exaggerated sensitivity to pain such as hyperalgesia, causalgia, and allodynia; acute pain; burn pain; atypical facial pain; neuropathic pain; back pain; complex regional pain syndrome I and II; arthritic pain; sports injury pain; dental pain; pain related to infection e.g. by HIV; post-chemotherapy pain; post-stroke pain; post-operative pain; neuralgia; osteoarthritis; conditions associated with visceral pain such as irritable bowel syndrome; eating disorders; diabetes; toxic and dysmetabolic disorders including cerebral anoxia, diabetic neuropathies and alcoholism; appetite, taste, eating, or drinking disorders; somatoform disorders including hypochondriasis; vomiting/nausea; emesis; gastric dyskinesia; gastric ulcers; Kallman's syndrome (anosmia); impaired glucose tolerance; intestinal motility dyskinesias; hypothalamic diseases; hypophysis diseases; hyperthermia syndromes, pyrexia, febrile seizures, idiopathic growth deficiency; dwarfism; gigantism; acromegaly; basophil adenoma; prolactinoma; hyperprolactinemia; brain tumors, adenomas; benign prostatic hypertrophy, prostate cancer; endometrial, breast, colon cancer; all types of testicular dysfunctions, fertility control; reproductive hormone abnormalities; hot flashes; hypothalamic hypogonadism, functional or psychogenic amenorrhea; urinary bladder incontinence; asthma; allergies; all types of dermatitis, acne and cysts, sebaceous gland dysfunctions; cardiovascular disorders; heart and lung diseases, acute and congestive heart failure; hypotension; hypertension; dyslipidemias, hyperlipidemias, insulin resistance; urinary retention; osteoporosis; angina pectoris; myocardial infarction; arrhythmias, coronary diseases, left ventricular hypertrophy; ischemic or haemorrhagic stroke; all types of cerebrovascular disorders including subarachnoid haemorrhage, ischemic and hemorrhagic stroke and vascular dementia; chronic renal failure and other renal diseases; gout; kidney cancer; urinary incontinence; and other diseases related to general orexin system dysfunctions.

Compounds of formula (I) and/or (Ia) are particularly suitable for use in the treatment of diseases or disorders selected from the group consisting of all types of sleep disorders, of stress-related syndromes, of psychoactive substance use and abuse, of cognitive dysfunctions in the healthy population and in psychiatric and neurologic disorders, of eating or drinking disorders.

Eating disorders may be defined as comprising metabolic dysfunction; dysregulated appetite control; compulsive obesities; emeto-bulimia or anorexia nervosa. Pathologically modified food intake may result from disturbed appetite (attraction or aversion for food); altered energy balance (intake vs. expenditure); disturbed perception of food quality (high fat or carbohydrates, high palatability); disturbed food availability (unrestricted diet or deprivation) or disrupted water balance. Drinking disorders include polydipsias in psychiatric disorders and all other types of excessive fluid intake. Sleep disorders include all types of parasomnias, insomnias, narcolepsy and other disorders of excessive sleepiness, sleep-related dystonias; restless leg syndrome; sleep apneas; jet-lag syndrome; shift-work syndrome, delayed or advanced sleep phase syndrome or insomnias related to psychiatric disorders. Insomnias are defined as comprising sleep disorders associated with aging; intermittent treatment of chronic insomnia; situational transient insomnia (new environment, noise) or short-term insomnia due to stress; grief; pain or illness. Insomnia also include stress-related syndromes including post-traumatic stress disorders as well as other types and subtypes of anxiety disorders such as generalized anxiety, obsessive compulsive disorder, panic attacks and all types of phobic anxiety and avoidance; psychoactive substance use, abuse, seeking and reinstatement are defined as all types of psychological or physical addictions and their related tolerance and dependence components. Cognitive dysfunctions include deficits in all types of attention, learning and memory functions occurring transiently or chronically in the normal, healthy, young, adult or aging population, and also occurring transiently or chronically in psychiatric, neurologic, cardiovascular and immune disorders.

In a further preferred embodiment of the invention compounds of formula (I) and/or (Ia) are particularly suitable for use in the treatment of diseases or disorders selected from the group consisting of sleep disorders that comprises all types of insomnias, narcolepsy and other disorders of excessive sleepiness, sleep-related dystonias, restless leg syndrome, sleep apneas, jet-lag syndrome, shift-work syndrome, delayed or advanced sleep phase syndrome or insomnias related to psychiatric disorders.

In another preferred embodiment of the invention compounds of formula (I) and/or (Ia) are particularly suitable for use in the treatment of diseases or disorders selected from the group consisting of cognitive dysfunctions that comprise deficits in all types of attention, learning and memory functions occurring transiently or chronically in the normal, healthy, young, adult or aging population, and also occurring transiently or chronically in psychiatric, neurologic, cardiovascular and immune disorders.

In another preferred embodiment of the invention compounds of formula (I) and/or (Ia) are particularly suitable for use in the treatment of diseases or disorders selected from the group consisting of eating disorders that comprise metabolic dysfunction; dysregulated appetite control; compulsive obesities; emeto-bulimia or anorexia nervosa.

In another preferred embodiment of the invention compounds of formula (I) and/or (Ia) are particularly suitable for use in the treatment of diseases or disorders selected from the group consisting of psychoactive substance use and abuse that comprise all types of psychological or physical addictions and their related tolerance and dependence components.

The compounds of formula (I) and/or (Ia) are useful for the treatment and/or prevention of the diseases mentioned herein.

Besides, any preferences indicated for the compounds of formula (I) (whether for the compounds themselves, salts thereof, compositions containing the compounds or salts thereof, uses of the compounds or salts thereof, etc.) apply mutatis mutandis to compounds of formula (Ia).

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10 °C to Y plus 10 °C, and preferably to an interval extending from Y minus 5 °C to Y plus 5 °C. Besides, the term "room temperature" (RT) as used herein refers to a temperature of about 25°C.

### Preparation of compounds of formula (I)

A further aspect of the invention is a process for the preparation of compounds of formula (I) and (Ia). Compounds according to formula (I) and (Ia) of the present invention can be prepared according to the general sequence of reactions outlined in the schemes below wherein A, B, X, n, R¹ and R² are as defined in the description for formula (I) and (Ia). Additional generic groups as used in the schemes below are defined as followed: R represents hydrogen or (C₁₋₄)alkyl; and R' represents hydrogen, (C₁₋₄)alkyl, (C₁₋₄)alkoxy, trifluoromethyl, cyano or halogen. The compounds obtained may also be converted into salts, especially pharmaceutically acceptable salts thereof in a manner known *per se.*

The compounds of formula (I) and (Ia) wherein X represents -NH-R¹ may be prepared starting from (+/-)-3-amino-1-N-Boc-piperidine or -pyrrolidine or from enantiomerically pure (R)-3-amino-1-N-Boc-piperidine or -pyrrolidine ((1), all commercially available) by reaction with the corresponding commercially available or well known 2-chloro-heteroaryl, 2-bromo-heteroaryl, or 2-trifluoromethanesulfonyl-heteroaryl derivative under basic conditions such as K₂CO₃ in presence of DIEA in a solvent such as xylene at reflux. These reaction conditions are especially successful in case of reactive heteroaryl-chlorides such as 2-chloro-pyrimidines or 2-chloro-pyridines, preferably substituted by electron-withdrawing substituents. Alternatively, the Buchwald-Hartwig method may be used for the above coupling reaction (for reaction conditions see the procedures given in eg. J. Med. Chem., 2007, 50, 3497-3514; J. F. Hartwig, "Modern Amination Methods", A. Ricci (Ed), Wiley-VCH Verlag GmbH, D-69469 Weinheim, 2000; ISBN 3-527-29976-9; Chapter 7, p. 195-262). The resulting amine (2) is transformed to compounds (3) by cleavage of the Boc protecting group under acidic conditions such as TFA in DCM followed by amide formation with the respective carboxylic acid B-A-CO₂H using standard amide coupling techniques such as PyBOP in presence of DIEA in a solvent such as DMF or TBTU in the presence of DIEA in a solvent such as MeCN (scheme 1).

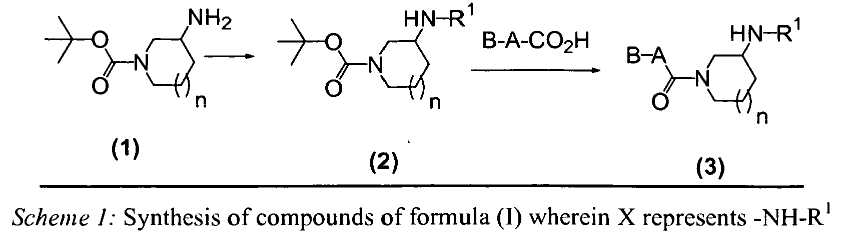

The compounds of formula (I) and (Ia), wherein X represents -NH-C(O)-R² may be prepared starting from (1) by reaction with the respective carboxylic acid derivative R²-CO₂H using standard amide coupling techniques as above. The resulting amide intermediates (4) are transformed to compounds (5) by cleavage of the Boc protecting group as described above, followed by amide formation with the respective carboxylic acid B-A-CO₂H (scheme 2) as described above.

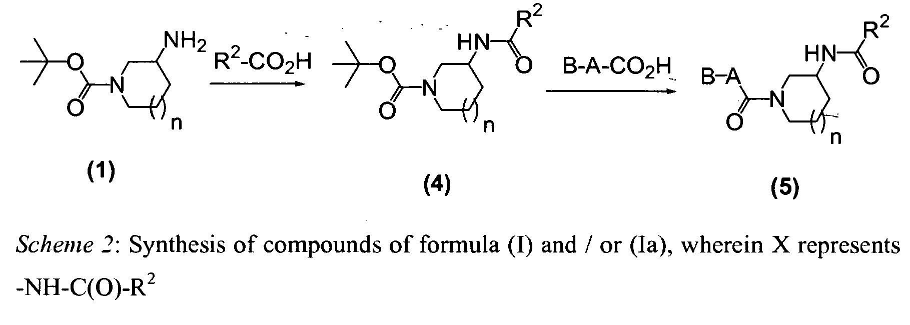

### Preparation of carboxylic acids B-A-CO₂H and R²-CO₂H

Carboxylic acid derivatives B-A-CO₂H wherein A represents a 2-cyclopropyl-thiazolyl derivative can be synthesised according to scheme 3.

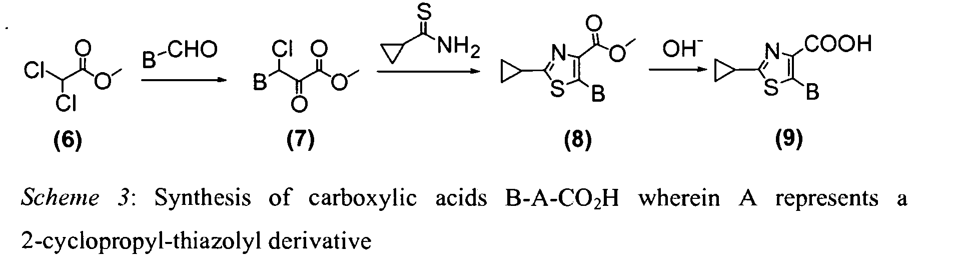

By reaction of methyl dichloroacetate (6) with commercially available benzaldehyde derivatives B-CHO in the presence of a base such as KO*t*Bu in an aprotic polar solvent such as THF at RT 3-chloro-2-oxo-propionic acid ester derivatives (7) are obtained (Hamamoto H. et al Tetrahedron Asymmetry 2000, 11, 4485-4497). Compounds of structure (7) can be transformed by reaction with cyclopropyl thioamide at RT in solvents such as MeCN to provide 2-cyclopropyl-thiazol-4-carboxylic acid ester derivatives (8) (US3282927). Saponification of the ester function using standard methodology, like treatment with a base such as NaOH in a solvent such as MeOH provides the corresponding 2-cyclopropyl-5-phenyl-thiazol-4-carboxylic acid derivatives (9). The respective benzaldehydes are commercially available or easily accessible via standard methodology from precursors like benzylalcohols or benzoic acids. The cyclopropyl-thioamide can be synthesized from commercially available cyclopropyl-carboxamide with *Lawesson's* reagent.

Carboxylic acid derivatives B-A-CO₂H, wherein B-A represents a biphen-2-yl derivative, are commercially available or can be synthesized according to scheme 4.

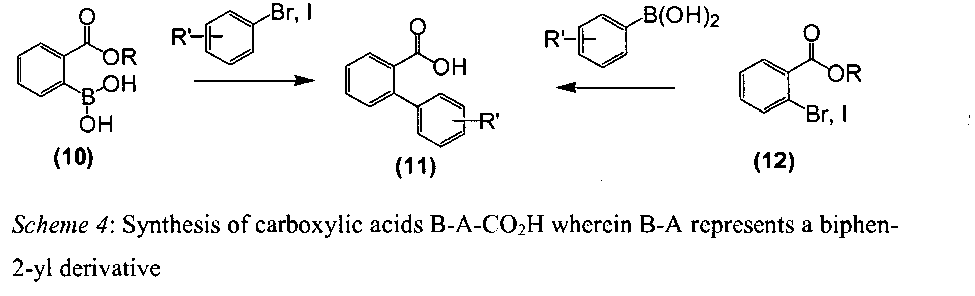

Reaction of commercially available (2-carboxyphenyl)-boronic acid derivatives (10) or esters thereof with commercially available phenyl-bromides or phenyl-iodides in presence of a catalyst such as Pd(PPh₃)₄ and a base such as Na₂CO₃ under heating in a solvent like toluene, dioxane, THF provides, after saponification, if needed, of the ester using well known methods, the corresponding biphenyl-2-carboxylic acid derivatives (11). Alternatively, reaction of commercially available 2-bromo-, or 2-iodo-benzoic acid derivatives (12), or esters thereof, with commercially available phenyl-boronic acid derivatives using the conditions described before provides the corresponding biphenyl-2-carboxylic acid derivatives (11).

Carboxylic acids of formula R²-CO₂H are commercially available or well known in the art (Lit. e.g. WO2001/96302; T. Eicher, S. Hauptmann "The chemistry of Heterocycles: Structure, Reactions, Syntheses, and Applications", 2nd Edition 2003, Wiley, ISBN 978-3-527-30720-3).

Derivatives of formula R²-CO₂H wherein R² is 2*H*-chromenyl or chromanyl may be for instance synthesised according to *scheme 5.*

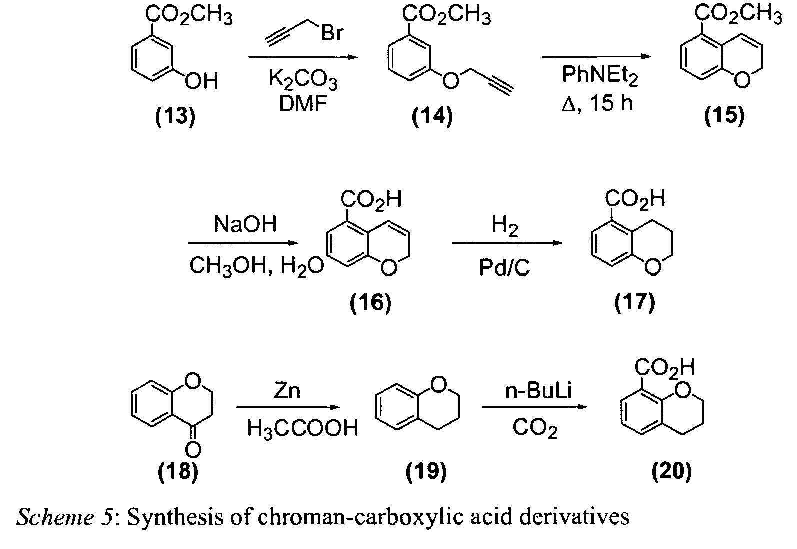

The synthesis of chroman-5-carboxylic acid derivatives may be started with the alkylation of 3-hydroxy-benzoic acid methyl ester (**13**; commercially available) with propargyl bromide in the presence of K₂CO₃ to give phenylether (**14**) which may be cyclised to the chromen derivative (**15**) by heating to reflux in N,N-diethylaniline. The carboxylic ester may be saponified by treatment of (**15**) with NaOH in MeOH and water and the obtained chromen derivative (**16**) may be hydrogenated to give the desired acid (**17**). The corresponding chroman-8-carboxylic acid derivatives may be synthesized by reduction of 4-chromanone (**18**; commercially available) with zinc in acetic acid and subsequent *ortho*-metalation of the intermediate chroman derivative (**19**) with n-BuLi and trapping with carbon dioxide to give the desired acid (**20**).

Whenever the compounds of formula (I) are obtained in the form of mixtures of enantiomers, the enantiomers can be separated using methods known to one skilled in the art: e.g. by formation and separation of diastereomeric salts or by HPLC over a chiral stationary phase such as a Regis Whelk-O1(R,R) (10 µm) column, a Daicel ChiralCel OD-H (5-10 µm) column, or a Daicel ChiralPak IA (10 µm) or AD-H (5 µm) column. Typical conditions of chiral HPLC are an isocratic mixture of eluent A (EtOH, in presence or absence of an amine such as triethylamine, diethylamine) and eluent B (hexane), at a flow rate of 0.8 to 150 mL/min.

### Experimental Section

**Abbrevations (as used herein and in the description above):**
- aq.: Aqueous
- Boc: *tert*-butoxycarbonyl
- BSA: Bovine serum albumine
- CHO: Chinese hamster ovary
- DCM: Dichloromethane
- DIEA: Disopropylethylamine
- DMF: *N,N*-dimethylformamide
- DMSO: Dimethyl sulfoxide
- EA: Ethyl acetate
- eq: Equivalent(s)
- ES: Electron spray
- ether: Diethylether
- FC: Flash chromatography on silica gel
- FCS: Foatal calf serum
- FLIPR: Fluorescent imaging plate reader
- h: Hour(s)
- HBSS: Hank's balanced salt solution
- HEPES: 4-(2-hydroxyethyl)-piperazine-1-ethanesulfonic acid
- HPLC: High performance liquid chromatography
- KOtBu: Potassium tert-butoxide
- LC: Liquid chromatography
- M: Molar(ity)
- MeCN: Acetonitrile
- MeOH: Methanol
- min: Minute(s)
- MS: Mass spectroscopy
- n-BuLi: n-Butyl lithium
- Ph: phenyl

- PyBOP: (Benzotriazole-lyloxy)-tripyrrolidinophosphonium-hexafluorophosphate
- i-PrOH: Isopropanol
- RT: Room temperature
- TBTU: 2-(1H-Benzotriazole-1-yl)-1,1,3,3-tetramethyluronium tetrafluoroborate
- tert: Tertiary
- THF: Tetrahydrofuran
- TFA: Trifluoroacetic acid
- TLC: Thin layer chromatography
- t_{R}: Retention time

### I-Chemistry

The following examples illustrate the preparation of biologically active compounds of the invention but do not at all limit the scope thereof.

All temperatures are stated in °C.

Compounds are characterized by:
LC-MS: Agilent 1100 series with DAD and MS detection (MS: Finnigan single quadrupole);
   columns (4.6x50 mm, 5 µm): Zorbax SB-AQ, Zorbax Extend C18 or Waters XBridge C 18;
   conditions (if not otherwise stated the acidic gradient is used): acidic: eluent A: MeCN, eluent B: TFA in water (0.4 mL/L), 5% to 95% CH₃CN, flow rate 4.5 mL/min; t_{R} is given in min.

Compounds are purified by FC, TLC or by preparative HPLC using RP-C₁₈ based columns with MeCN/water gradients and formic acid or ammonia additives.

### A. Preparation of precursors and intermediates:

### A.1 Synthesis of 2-cyclopropyl-thiazole-4-carboxylic acid derivatives

### A.1.1 Synthesis of 3-chloro-2-oxo-propionic ester derivatives

### (general procedure)

A solution of the respective aldehyde B-CHO (338 mmol, 1.0 eq) and methyl dichloroacetate (338 mmol, 1.0 eq) in THF (100 mL) is added dropwise to a cold (-60°C) suspension of KOtBu (335 mmol, 1.0 eq) in THF (420 mL). After 4 h the mixture is allowed to reach RT, stirred over night and concentrated in vacuo. DCM and ice-cold water are added, the layers are separated and the aqueous layer is extracted twice with DCM. The combined organic layers are washed with ice-cold water and brine, dried over MgSO₄ and concentrated in vacuo to give the desired 2-oxo-propionic acid ester which is used without further purification.
**3-chloro-2-oxo-3-m-tolyl-propionic acid methyl ester**
prepared by reaction of 3-methyl-benzaldehyde with methyl dichloroacetate.
**3-chloro-2-oxo-3-p-tolyl-propionic acid methyl ester**
prepared by reaction of 4-methyl-benzaldehyde with methyl dichloroacetate.
**3-chloro-3-(3-fluoro-phenyl)-2-oxo-propionic acid methyl ester**
prepared by reaction of 3-fluoro-benzaldehyde with methyl dichloroacetate.
**3-chloro-3-(4-fluoro-phenyl)-2-oxo-propionic acid methyl ester**
prepared by reaction of 4-fluoro-benzaldehyde with methyl dichloroacetate.
**3-chloro-3-(3-trifluoromethyl-phenyl)-2-oxo-propionic acid methyl ester**
prepared by reaction of 3-trifluoromethyl-benzaldehyde with methyl dichloro-acetate.
**3-chloro-3-(2-fluoro-phenyl)-2-oxo-propionic acid methyl ester**
prepared by reaction of 2-fluoro-benzaldehyde with methyl dichloro-acetate.
**3-chloro-3-(3-methoxy-phenyl)-2-oxo-propionic acid methyl ester**
prepared by reaction of 3-methoxy-benzaldehyde with methyl dichloro-acetate.
**3-chloro-3-(3-fluoro-4-methyl-phenyl)-2-oxo-propionic acid methyl ester**
prepared by reaction of 3,4-dimethyl-benzaldehyde with methyl dichloro-acetate.
**3-chloro-3-phenyl-2-oxo-propionic acid methyl ester**
prepared by reaction of benzaldehyde with methyl dichloro-acetate.
**3-chloro-3-(3-bromo-phenyl)-2-oxo-propionic acid methyl ester**
prepared by reaction of 3-bromo-benzaldehyde with methyl dichloro-acetate.
**3-chloro-3-(4-cyano-phenyl)-2-oxo-propionic acid methyl ester**
prepared by reaction of 4-cyano-benzaldehyde with methyl dichloro-acetate.
**3-chloro-3-(3-fluoro-5-trifluoromethyl-phenyl)-2-oxo-propionic acid methyl ester**
prepared by reaction of 3-fluoro-5-trifluoromethyl-benzaldehyde with methyl dichloro-acetate.

### A.1.2 Synthesis of 2-cyclopropyl-thiazole-4-carboxylic acid methyl ester derivatives

### (general procedure)

A solution of cyclopropanecarbothioamide (132 mmol, 1.0 eq) in MeCN (250 mL) is added to a mixture of the respective 2-oxo-propionic acid ester (132 mmol, 1.0 eq) and molecular sieves (4Å, 12 g) in MeCN (60 mL). After stirring for 5 h the mixture is cooled in an ice-bath and the obtained precipitate is filtered off. The residue is washed with cold MeCN, dried, dissolved in MeOH (280 mL) and stirred at 50°C for 6 h. The solvents are removed in vacuo to give the desired thiazole derivatives as a white solid.
**2-cyclopropyl-5-m-tolyl-thiazole-4-carboxylic acid methyl ester**
prepared by reaction of 3-chloro-2-oxo-3-m-tolyl-propionic acid methyl ester with cyclopropanecarbothioamide. LC-MS: t_{R} = 0.99 min; [M+H]⁺ = 274.27.
**2-cyclopropyl-5-p-tolyl-thiazole-4-carboxylic acid methyl ester**
prepared by reaction of 3-chloro-2-oxo-3-p-tolyl-propionic acid methyl ester with cyclopropanecarbothioamide. LC-MS: t_{R} = 1.04 min; [M+H]⁺ = 274.36.
**5-(3-fluoro-phenyl)-2-cyclopropyl-thiazole-4-carboxylic acid methyl ester**
prepared by reaction of 3-chloro-3-(3-fluoro-phenyl)-2-oxo-propionic acid methyl ester with cyclopropanecarbothioamide. LC-MS: t_{R} = 1.02 min; [M+H]⁺ = 278.04.
**5-(4-fluoro-phenyl)-2-cyclopropyl-thiazole-4-carboxylic acid methyl ester**
prepared by reaction of 3-chloro-3-(4-fluoro-phenyl)-2-oxo-propionic acid methyl ester with cyclopropanecarbothioamide. LC-MS: t_{R} = 1.01 min; [M+H]⁺ = 278.32.
**2-cyclopropyl-5-(3-trifluoromethyl-phenyl)-thiazole-4-carboxylic acid methyl ester**
prepared by reaction of 3-chloro-3-(3-trifluoromethyl-phenyl)-2-oxo-propionic acid methyl ester with cyclopropanecarbothioamide. LC-MS: t_{R} = 1.07 min; [M+H]⁺ = 328.23.
**2-cyclopropyl-5-(2-fluoro-phenyl)-thiazole-4-carboxylic acid methyl ester**
prepared by reaction of 3-chloro-3-(2-fluoro-phenyl)-2-oxo-propionic acid methyl ester with cyclopropanecarbothioamide. LC-MS: t_{R} = 0.95 min; [M+H]⁺ = 278.27.
**2-cyclopropyl-5-(3-methoxy-phenyl)-thiazole-4-carboxylic acid methyl ester**
prepared by reaction of 3-chloro-3-(3-methoxy-phenyl)-2-oxo-propionic acid methyl ester with cyclopropanecarbothioamide. LC-MS: t_{R} = 0.96 min; [M+H]⁺ = 290.30.
**5-(3-fluoro-4-methyl-phenyl)-2-cyclopropyl-thiazole-4-carboxylic acid methyl ester**
prepared by reaction of 3-chloro-3-(3-fluoro-4-methyl-phenyl)-2-oxo-propionic acid methyl ester with cyclopropanecarbothioamide. LC-MS: t_{R} = 1.02 min; [M+H]⁺ = 292.07.
**5-phenyl-2-cyclopropyl-thiazole-4-carboxylic acid methyl ester**
prepared by reaction of 3-chloro-3 -phenyl-2-oxo-propionic acid methyl ester with cyclopropanecarbothioamide. LC-MS: t_{R} = 0.99 min; [M+H]⁺ = 260.45.
**5-(3-bromo-phenyl)-2-cyclopropyl-thiazole-4-carboxylic acid methyl ester**
prepared by reaction of 3-chloro-3-(3-bromo-phenyl)-2-oxo-propionic acid methyl ester with cyclopropanecarbothioamide. LC-MS: t_{R} = 1.03 min; [M+H]⁺ = 339.91.
**5-(4-cyano-phenyl)-2-cyclopropyl-thiazole-4-carboxylic acid methyl ester**
prepared by reaction of 3-chloro-3-(4-cyano-phenyl)-2-oxo-propionic acid methyl ester with cyclopropanecarbothioamide. LC-MS: t_{R} = 1.00 min; [M+H]⁺ = 285.02.
**5-(3-fluoro-5-trifluoromethyl-phenyl)-2-methyl-thiazole-4-carboxylic acid methyl ester**
prepared by reaction of 3-chloro-3-(3-fluoro-5-trifluoromethyl-phenyl)-2-oxo-propionic acid methyl ester with cyclopropanecarbothioamide. LC-MS: t_{R} = 1.09 min; [M+H]⁺ = 345.99.

### A.1.3 Synthesis of thiazole-4-carboxylic acid derivatives

### (general procedure)

A solution of the respective thiazole-4-carboxylic acid ester derivative (96.2 mmol) in a mixture of THF (150 mL) and i-PrOH (50 mL) is treated with an aq. NaOH solution (1.0 M, 192 mL). After stirring for several h a white suspension is formed and the organic volatiles are removed in vacuo. The remaining mixture is diluted with water (100 mL), cooled in an ice-bath and made acidic (pH = 3-4) by addition of aq. HCl solution (1.0 M). The suspension is filtered and the residue is washed with cold water. After drying the desired acid is obtained as a white solid.
**2-cyclopropyl-5-m-tolyl-thiazole-4-carboxylic acid**
prepared by hydrolysis of 2-cyclopropyl-5-m-tolyl-thiazole-4-carboxylic acid methyl ester. LC-MS: t_{R} = 0.92 min; [M+H]⁺ = 260.02.
**2-cyclopropyl-5-p-tolyl-thiazole-4-carboxylic acid**
prepared by hydrolysis of 2-cyclopropyl-5-p-tolyl-thiazole-4-carboxylic acid methyl ester. LC-MS: t_{R} = 0.91 min; [M+H]⁺ = 260.03.
**5-(3-fluoro-phenyl)-2-cyclopropyl-thiazole-4-carboxylic acid**
prepared by hydrolysis of 5-(3-fluoro-phenyl)-2-cyclopropyl-thiazole-4-carboxylic acid methyl ester. LC-MS: t_{R} = 0.92 min; [M+H]⁺ = 264.01.
**5-(4-fluoro-phenyl)-2-cyclopropyl-thiazole-4-carboxylic acid**
prepared by hydrolysis of 5-(4-fluoro-phenyl)-2-cyclopropyl-thiazole-4-carboxylic acid methyl ester. LC-MS: t_{R} = 0.88 min; [M+H]⁺ = 263.99.
**2-cyclopropyl-5-(3-trifluoromethyl-phenyl)-thiazole-4-carboxylic acid**
prepared by hydrolysis of 2-cyclopropyl-5-(3-trifluoromethyl-phenyl)-thiazole-4-carboxylic acid methyl ester. LC-MS: t_{R} = 1.00 min; [M+H]⁺ = 314.27.
**2-cyclopropyl-5-(2-fluoro-phenyl)-thiazole-4-carboxylic acid**
prepared by hydrolysis of 2-cyclopropyl-5-(2-fluoro-phenyl)-thiazole-4-carboxylic acid methyl ester. LC-MS: t_{R} = 0.91 min; [M+H]⁺ = 264.27.
**2-cyclopropyl-5-(3-methoxy-phenyl)-thiazole-4-carboxylic acid**
prepared by hydrolysis of 2-cyclopropyl-5-(3-methoxy-phenyl)-thiazole-4-carboxylic acid methyl ester. LC-MS: t_{R} = 0.87 min; [M+H]⁺ = 276.27.
**5-(3-fluoro-4-methyl-phenyl)-2-cyclopropyl-thiazole-4-carboxylic acid**
prepared by hydrolysis of 5-(3-fluoro-4-methyl-phenyl)-2-cyclopropyl-thiazole-4-carboxylic acid methyl ester. LC-MS: t_{R} = 0.97 min; [M+H]⁺ = 278.06.
**5-phenyl-2-cyclopropyl-thiazole-4-carboxylic acid**
prepared by hydrolysis of 5-phenyl-2-cyclopropyl-thiazole-4-carboxylic acid methyl ester. LC-MS: t_{R} = 0.91 min; [M+H]⁺ = 246.39.
**5-(3-bromo-phenyl)-2-cyclopropyl-thiazole-4-carboxylic acid**
prepared by hydrolysis of 5-(3-bromo-phenyl)-2-cyclopropyl-thiazole-4-carboxylic acid methyl ester. LC-MS: t_{R} = 0.97 min; [M+H]⁺ = 323.80.
**5-(4-cyno-phenyl)-2-cyclopropyl-thiazole-4-carboxylic acid**
prepared by hydrolysis of 5-(4-cyno-phenyl)-2-cyclopropyl-thiazole-4-carboxylic acid methyl ester. LC-MS: t_{R} = 0.90 min; [M+H]⁺ = 270.99.
**5-(3-fluoro-5-trifluoromethyl-phenyl)-2-methyl-thiazole-4-carboxylic acid**
prepared by hydrolysis of 5-(3-fluoro-5-trifluoromethyl-phenyl)-2-methyl-thiazole-4-carboxylic acid methyl ester. LC-MS: t_{R} = 1.01 min; [M+H]⁺ = 332.03.

### Preparation of Examples

### B. Example 1: (R)-(3',4'-Dimethyl-biphenyl-2-yl)-[3-(4-trifluoromethyl-pyrimidin-2-ylamino)-piperidin-1-yl]-methanone

### B.1.1 Synthesis of (R)-[1-(3',4'-Dimethyl-biphenyl-2-carbonyl)-piperidin-3-yl]-carbamic acid tert-butyl ester

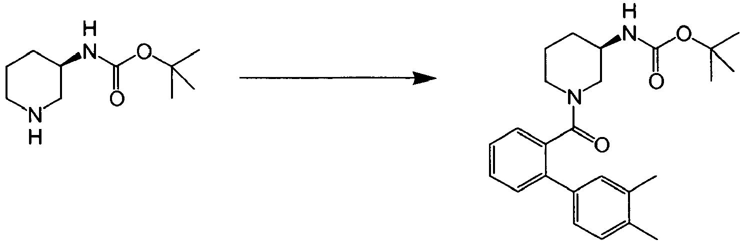

3',4'-Dimethyl-biphenyl-2-carboxylic acid (1.13 g, 4.99 mmol) was dissolved in MeCN (40 ml) followed by the addition of TBTU (1.76 g, 5.49 mmol) and DIEA (0.97 g, 7.49 mmol). Stirring was continued for 15 min at RT followed by the addition of (R)-3-(tert-butoxycarbonylamino)piperidine (1.02 g, 4.99 mmol). Stirring at RT was continued for 1 h. The reaction mixture was poured onto 1 M aq. HCl (200 ml). The organic layer was separated, the solvent was evaporated under reduced pressure and the residue dried at HV to give 1.8 g of (R)-[1-(3',4'-Dimethyl-biphenyl-2-carbonyl)-piperidin-3-yl]-carbamic acid tert-butyl ester as a sticky glue.
LC-MS: t_{R} = 1.01 min; [M+H]⁺ = 409.08.

### B.1.2 Synthesis of (R)-(3-Amino-piperidin-1-yl)-(3',4'-dimethyl-biphenyl-2-yl)-methanone

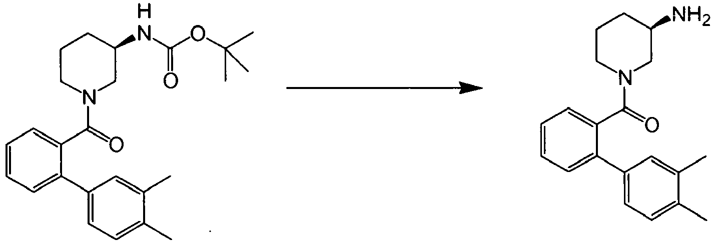

(R)-[1-(3',4'-Dimethyl-biphenyl-2-carbonyl)-piperidin-3-yl]-carbamic acid tert-butyl ester was dissolved in dioxane (12 ml) followed by the addition of a solution of HCl in dioxane (4M, 12 ml). Stirring was continued for 1 h at RT. The solvent was evaporated under reduced pressure and the residue was dried at HV to give 1.8 g of (R)-(3-Amino-piperidin-1-yl)-(3',4'-dimethyl-biphenyl-2-yl)-methanone dihydrochloride. LC-MS: t_{R} = 0.72 min; [M+H]⁺ = 295.14.

### B.1.3 Synthesis of (R)-(3',4'-Dimethyl-biphenyl-2-yl)-[3-(4-trifluoromethyl-pyrimidin-2-ylamino)-piperidin-1-yl]-methanone

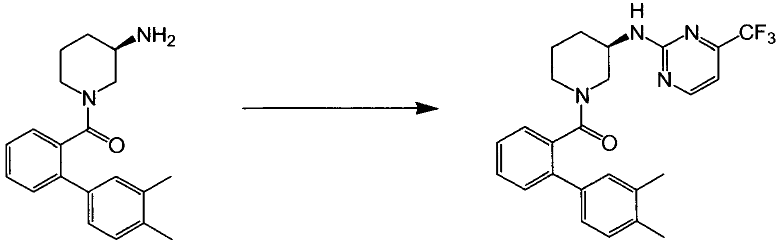

2-Chloro-4-trifluoromethyl-pyrimidine (31.6 mg, 0.173 mmol), anhydrous potassium carbonate (130.5 mg, 0.944 mmol) and (R)-(3-Amino-piperidin-1-yl)-(3',4'-dimethylbiphenyl-2-yl)-methanone dihydrochloride (60 mg, 0.157 mmol) were dissolved in o-xylene (1 ml) and DIEA (0.081 ml) and heated to 145°C for 12 h under an inert atmosphere. The reaction mixture was cooled to RT followed by careful addition of 2M aq. HCl (3 ml). The product was extracted with ether. The solvent was evaporated and the residue was purified by preparative TLC (silicagel, 0.5 mm; EA / heptane = 3 / 2) to give 42 mg of (R)-(3',4'-Dimethyl-biphenyl-2-yl)-[3-(4-trifluoromethyl-pyrimidin-2-ylamino)-piperidin-1-yl]-methanone.
LC-MS: t_{R} = 1.11 min; [M+H]⁺ = 455.08.

Examples 2 to 10 were prepared according to the description for the preparation of example 1:

### Example 2: (R)-[3-(4,6-Dimethoxy-pyrimidin-2-ylamino)-piperidin-1-yl]-(3',4'-dimethyl-biphenyl-2-yl)-methanone

LC-MS: t_{R} = 1.05 min; [M+H]⁺ = 447.06.

### Example 3: (R)-(3'-Methyl-biphenyl-2-yl)-[3-(4-trifluoromethyl-pyrimidin-2-ylamino)-piperidin-1-yl]-methanone

LC-MS: t_{R} = 1.08 min; [M+H]⁺ = 441.14.

### Example 4: (R)-[3-(4,6-Dimethoxy-pyrimidin-2-ylamino)-piperidin-1-yl]-(3'-methyl-biphenyl-2-yl)-methanone

LC-MS: t_{R} = 1.02 min; [M+H]⁺ = 433.17.

### Example 5: (R)-[3-(6,7-Difluoro-quinoxalin-2-ylamino)-piperidin-1-yl]-(3',4'-dimethyl-biphenyl-2-yl)-methanone

LC-MS: t_{R} = 1.12 min; [M+H]⁺ = 473.38.

### Example 6: (R)-(3',4'-Dimethyl-biphenyl-2-yl)-[3-(quinoxalin-2-ylamino)-piperidin-1-yl]-methanone

LC-MS: t_{R} = 1.05 min; [M+H]⁺ = 437.42.

### Example 7: (R)-(3',4'-Dimethyl-biphenyl-2-yl)-[3-(5-methylsulfanyl-pyrimidin-2-ylamino)-piperidin-1-yl]-methanone

LC-MS: t_{R} = 1.07 min; [M+H]⁺ = 433.35.

### Example 8: (R)-2-[1-(3',4'-Dimethyl-biphenyl-2-carbonyl)-piperidin-3-ylamino]-6-methyl-pyrimidine-4-carboxylic acid methyl ester

LC-MS: t_{R} = 1.04 min; [M+H]⁺ = 459.48.

### Example 9: (R)-[3-(Benzothiazol-2-ylamino)-piperidin-1-yl]-(3',4'-dimethyl-biphenyl-2-yl)-methanone

LC-MS: t_{R} = 0.99 min; [M+H]⁺ = 442.40.

### Example 10: (R)-[3-(1H-Benzoimidazol-2-ylamino)-piperidin-1-yl]-(3',4'-dimethyl-biphenyl-2-yl)-methanone

LC-MS: t_{R} = 0.93 min; [M+H]⁺ = 425.26.

### C. Example 11: (R)-[2-Cyclopropyl-5-m-tolyl-thiazol-4-yl]-[3-(4-trifluoromethyl-pyrimidin-2-ylamino)-piperidin-1-yl]-methanone

### C.1.1. Synthesis of (R)-[1-(2-Cyclopropyl-5-m-tolyl-thiazole-4-carbonyl)-piperidin-3-yl]-carbamic acid tert-butyl ester

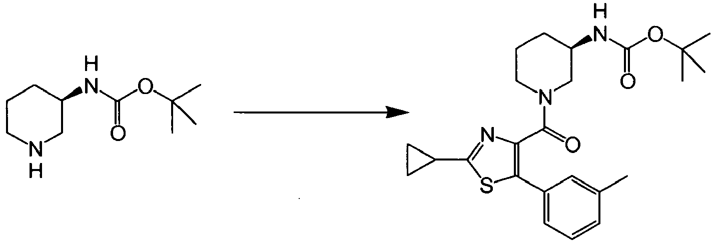

2-Cyclopropyl-5-m-tolyl-thiazole-4-carboxylic acid (1.62 g, 4.99 mmol) was dissolved in acetonitrile followed by the addition of TBTU (1.76 g, 5.49 mmol) and DIEA (3.23 g, 24.96 mmol). Stirring at RT was continued for 15 min. (R)-3-(tert-butoxycarbonylamino)piperidine 1.02 g, 4.99 mmol) was added to the reaction mixture and stirring at RT was continued for 2h. The reaction mixture was concentrated under reduced pressure and 1M aq. HCl solution (100 ml) was added to the residue. The product was extracted with ethyl acetate (2x 100 ml). The combined organic layers were dried over magnesium sulphate, filtered and concentrated under reduced pressure. The residue was purified by FC (ethyl acetate) to give 1.94 g of (R)-{1-[2-Cyclopropyl-5-m-tolyl-thiazole-4-carbonyle]-piperidin-3-yl}-carbamic acid tert-butyl ester. LC-MS: t_{R} = 1.08 min; [M+H]⁺ = 442.05.

### C.1.2. Synthesis of (R)-(3-Amino-piperidin-1-yl)-(2-cyclopropyl-5-m-tolyl-thiazol-4-yl)-methanone

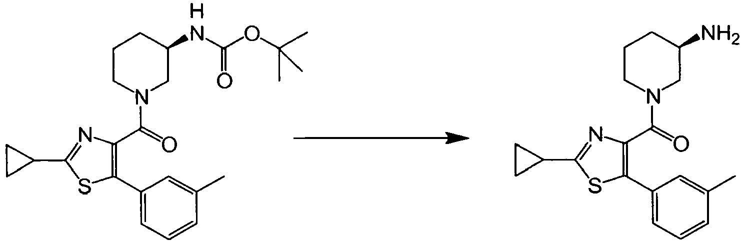

(R)-{1-[2-Cyclopropyl-5-m-tolyl-thiazole-4-carbonyl]-piperidin-3-yl}-carbamic acid tert-butyl ester 1.94 g, 4.39 mmol) was dissolved in dioxane (10 ml) followed by the addition of a solution of HCl in dioxane (4M, 11.2 ml). Stirring was continued for 1 h at RT. The solvent was evaporated under reduced pressure and the residue was dried at HV to give 1.88 g of (R)-(3-Amino-piperidin-1-yl)-[2-cyclopropyl-5-m-tolyl-thiazol-4-yl]-methanone dihydrochloride. LC-MS: t_{R} = 0.79 min; [M+H]⁺ = 342.08.

### C.1.3. Synthesis of (R)-(2-Cyclopropyl-5-m-tolyl-thiazol-4-yl)-[3-(4-trifluoromethyl-pyrimidin-2-ylamino)-piperidin-1-yl]-methanone

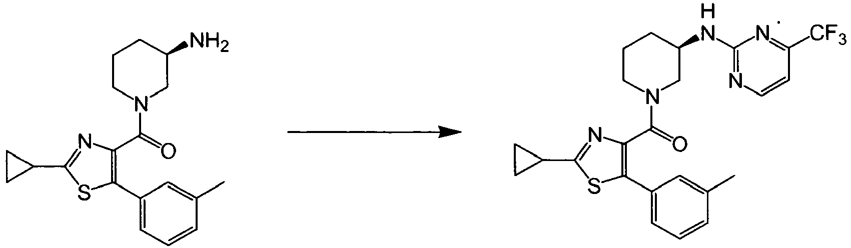

(R)-(3-Amino-piperidin-1-yl)-[2-cyclopropyl-5-m-tolyl-thiazol-4-yl]-methanone dihydrochloride (80 mg, 0.193 mmol), was dissolved in o-xylene (0.5 ml) followed by the addition of DIEA (87 mg, 0.67 mmol) and anhydrous potassium carbonate (173 mg, 1.25 mmol) and 2-chloro-4-trifluoromethyl-pyrimidine (107 mg, 0.579 mmol). The reaction mixture was heated to 145°C for 16 h. After cooling to RT, ether (2 ml) and 1M aq. HCl-solution (2 ml) were added. The organic layer was separated and the aq. layer was extracted again with ether. The combined organic layers were concentrated in vacuo and the residue was purified by TLC (silicagel, 0.5 mm; EA 100%) to give 29 mg of (R)-[2-Cyclopropyl-5-m-tolyl-thiazol-4-yl]-[3-(4-trifluoromethyl-pyrimidin-2-ylamino)-piperidin-1-yl]-methanone.

LC-MS: t_{R} = 1.11 min; [M+H]⁺ = 488.51.

Examples 12 to 14 were prepared according to the description for the preparation of example 11:

### Example 12: (R)-(2-Cyclopropyl-5-m-tolyl-thiazol-4-yl)-[3-(4,6-dimethoxy-pyrimidin-2-ylamino)-piperidin-1-yl]-methanone

LC-MS: t_{R} = 1.04 min; [M+H]⁺ = 480.05.

### Example 13: (R)-[2-Cyclopropyl-5-(4-fluoro-phenyl)-thiazol-4-yl]-[3-(4-trifluoromethyl-pyrimidin-2-ylamino)-piperidin-1-yl]-methanone

LC-MS: t_{R} = 1.09 min; [M+H]⁺ = 492.35.

### Example 14: (R)-[2-Cyclopropyl-5-(4-fluoro-phenyl)-thiazol-4-yl]-[3-(4,6-dimethoxy-pyrimidin-2-ylamino)-piperidin-1-yl]-methanone

LC-MS: t_{R} = 1.03 min; [M+H]⁺ = 484.43.

### D. Example 15: Synthesis of (R)-1-Methyl-1H-indazole-3-carboxylic acid [1-(2-cyclopropyl-5-m-tolyl-thiazole-4-carbonyl)-piperidin-3-yl]-amide

### D.1.1. (R)-1-Methyl-1H-indazole-3-carboxylic acid [1-(2-cyclopropyl-5-m-tolyl-thiazole-4-carbonyl)-piperidin-3-yl]-amide

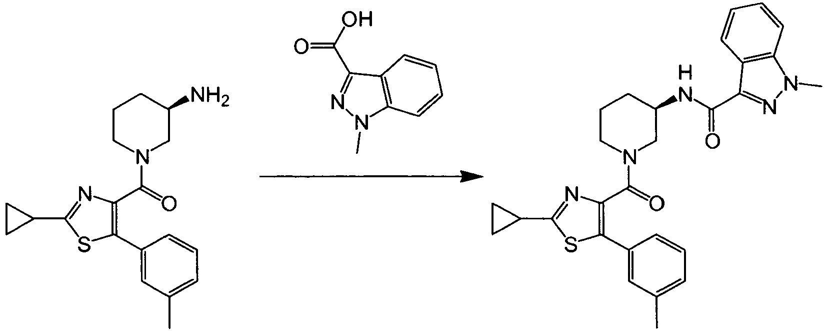

1-Methyl-1H-indazole-3-carboxylic acid (21.1 mg, 0.12 mmol) was dissolved in DMF (1 ml) followed by the addition of TBTU (42.4 mg, 0.132 mmol) and DIEA (77.6 mg, 0.60 mmol). Stirring at RT was continued for 15 min. (R)-(3-Amino-piperidin-1-yl)-(2-cyclopropyl-5-m-tolyl-thiazol-4-yl)-methanone (50 mg, 0.12 mmol) was added and stirring at RT was continued for 16h followed by the addition of formic acid (0.25 ml) and direct purification of the reaction mixture via preparative HPLC to give 17.5 mg of (R)-1-Methyl-1H-indazole-3-carboxylic acid [1-(2-cyclopropyl-5-m-tolyl-thiazole-4-carbonyl)-piperidin-3-yl]-amide. LC-MS: t_{R} = 1.07 min; [M+H]⁺ = 499.99.

### (Precursors were prepared according to procedures described above)

Examples 16 to 46 were prepared according to the description for the preparation of example 15:

### Example 16: (R)-2,3-Dihydro-thieno[3,4-b][1,4]dioxine-5-carboxylic acid [1-(2-cyclopropyl-5-m-tolyl-thiazole-4-carbonyl)-piperidin-3-yl]-amide

LC-MS: t_{R} = 1.05 min; [M+H]⁺ = 509.02.

### Example 17: (R)-imidazo[1,2-a]pyridine-3-carboxylic acid [1-(2-cyclopropyl-5-m-tolyl-thiazole-4-carbonyl)-piperidin-3-yl]-amide

LC-MS: t_{R} = 0.85 min; [M+H]⁺ = 486.02.

### Example 18: (R)-Benzo[d]isoxazole-3-carboxylic acid [1-(2-cyclopropyl-5-m-tolyl-thiazole-4-carbonyl)-piperidin-3-yl]-amide

LC-MS: t_{R} = 1.07 min; [M+H]⁺ = 486.94.

### Example 19: (R)-3-Chloro-N-[1-(2-cyclopropyl-5-m-tolyl-thiazole-4-carbonyl)-piperidin-3-yl]-2-methyl-benzamide

LC-MS: t_{R} = 1.09 min; [M+H]⁺ = 493.95.

### Example 20: (R)-N-[1-(2-Cyclopropyl-5-m-tolyl-thiazole-4-carbonyl)-piperidin-3-yl]-3-fluoro-2-methyl-benzamide

LC-MS: t_{R} = 1.07 min; [M+H]⁺ = 477.98.

### Example 21: (R)-N-[1-(2-Cyclopropyl-5-m-tolyl-thiazole-4-carbonyl)-piperidin-3-yl]-2,3-dimethyl-benzamide

LC-MS: t_{R} = 1.08 min; [M+H]⁺ = 474.02.

### Example 22: (R)-2,3-Dihydro-benzo[1,4]dioxine-5-carboxylic acid [1-(2-cyclopropyl-5-m-tolyl-thiazole-4-carbonyl)-piperidin-3-yl]-amide

LC-MS: t_{R} = 1.07 min; [M+H]⁺ = 503.97.

### Example 23: (R)-1-Methyl-1H-indole-3-carboxylic acid [1-(2-cyclopropyl-5-m-tolyl-thiazole-4-carbonyl)-piperidin-3-yl]-amide

LC-MS: t_{R} = 1.06 min; [M+H]⁺ = 499.01.

### Example 24: (R)-2-Ethyl-5-methyl-2H-pyrazole-3-carboxylic acid [1-(2-cyclopropyl-5-m-tolyl-thiazole-4-carbonyl)-piperidin-3-yl]-amide

LC-MS: t_{R} = 1.02 min; [M+H]⁺ = 478.03.

### Example 25: (R)-Quinoline-8-carboxylic acid [1-(2-cyclopropyl-5-m-tolyl-thiazole-4-carbonyl)-piperidin-3-yl]-amide

LC-MS: t_{R} = 1.04 min; [M+H]⁺ = 496.99.

### Example 26: (R)-Isoquinoline-1-carboxylic acid [1-(2-cyclopropyl-5-m-tolyl-thiazole-4-carbonyl)-piperidin-3-yl]-amide

LC-MS: t_{R} = 1.07 min; [M+H]⁺ = 497.02.

### Example 27: (R)-2,5-Dimethyl-2H-pyrazole-3-carboxylic acid [1-(2-cyclopropyl-5-m-tolyl-thiazole-4-carbonyl)-piperidin-3-yl]-amide

LC-MS: t_{R} = 1.01 min; [M+H]⁺ = 464.03.

### Example 28: (R)-2,3-Dihydro-thieno[3,4-b][1,4]dioxine-5-carboxylic acid {1-[2-cyclopropyl-5-(4-fluoro-phenyl)-thiazole-4-carbonyl]-piperidin-3-yl}-amide

LC-MS: t_{R} = 1.02 min; [M+H]⁺ = 513.86.

### Example 29: (R)-Imidazo[1,2-a]pyridine-3-carboxylic acid {1-[2-cyclopropyl-5-(4-fluoro-phenyl)-thiazole-4-carbonyl]-piperidin-3-yl}-amide

LC-MS: t_{R} = 0.84 min; [M+H]⁺ = 489.95.

### Example 30: (R)-Benzo[d]isoxazole-3-carboxylic acid {1-[2-cyclopropyl-5-(4-fluoro-phenyl)-thiazole-4-carbonyl]-piperidin-3-yl}-amide

LC-MS: t_{R} = 1.05 min; [M+H]⁺ = 490.95.

### Example 31: (R)-2,3-Dihydro-benzo[1,4]dioxine-5-carboxylic acid {1-[2-cyclopropyl-5-(4-fluoro-phenyl)-thiazole-4-carbonyl]-piperidin-3-yl}-amide

LC-MS: t_{R} = 1.04 min; [M+H]⁺ = 507.95.

### Example 32: (R)-1-Methyl-1H-indole-3-carboxylic acid {1-[2-cyclopropyl-5-(4-fluoro-phenyl)-thiazole-4-carbonyl]-piperidin-3-yl}-amide

LC-MS: t_{R} = 1.04 min; [M+H]⁺ = 502.98.

### Example 33: (R)-Isoquinoline-1-carboxylic acid {1-[2-cyclopropyl-5-(4-fluoro-phenyl)-thiazole-4-carbonyl]-piperidin-3-yl}-amide

LC-MS: t_{R} = 1.05 min; [M+H]⁺ = 500.96.

### Example 34: (R)-1-Methyl-1H-indazole-3-carboxylic acid [1-(3'-methyl-biphenyl-2-carbonyl)-piperidin-3-yl]-amide

LC-MS: t_{R} = 1.07 min; [M+H]⁺ = 453.46.

### Example 35: (R)-1-Methyl-1H-indole-3-carboxylic acid [1-(3'-methyl-biphenyl-2-carbonyl)-piperidin-3-yl]-amide

LC-MS: t_{R} = 1.05 min; [M+H]⁺ = 452.56.

### Example 36: (R)-1H-Pyrrolo[2,3-b]pyridine-4-carboxylic acid [1-(3'-methyl-biphenyl-2-carbonyl)-piperidin-3-yl]-amide

LC-MS: t_{R} = 0.92 min; [M+H]⁺ = 439.31.

### Example 37: (R)-2-Ethyl-5-methyl-2H-pyrazole-3-carboxylic acid [1-(3'-methyl-biphenyl-2-carbonyl)-piperidin-3-yl]-amide

LC-MS: tR = 1.01 min; [M+H]⁺ = 431.47.

### Example 38: (R)-Quinoline-8-carboxylic acid [1-(3'-methyl-biphenyl-2-carbonyl)-piperidin-3-yl]-amide

LC-MS: t_{R} = 1.03 min; [M+H]⁺ = 450.50.

### Example 39: (R)-1H-Pyrrolo[2,3-b]pyridine-5-carboxylic acid [1-(3'-methyl-biphenyl-2-carbonyl)-piperidin-3-yl]-amide

LC-MS: t_{R} = 0.95 min; [M+H]⁺ = 439.37.

### Example 40: (R)-1-Methyl-1H-indazole-3-carboxylic acid [1-(3',4'-dimethyl-biphenyl-2-carbonyl)-piperidin-3-yl]-amide

LC-MS: t_{R} = 1.09 min; [M+H]⁺ = 467.59.

### Example 41: (R)-1-Methyl-1H-indole-3-carboxylic acid [1-(3',4'-dimethyl-biphenyl-2-carbonyl)-piperidin-3-yl]-amide

LC-MS: t_{R} = 1.07 min; [M+H]⁺ = 466.49.

### Example 42: (R)-1H-Pyrrolo[2,3-b]pyridine-4-carboxylic acid [1-(3',4'-dimethyl-biphenyl-2-carbonyl)-piperidin-3-yl]-amide

LC-MS: t_{R} = 0.95 min; [M+H]⁺ = 453.49.

### Example 43: (R)-2-Ethyl-5-methyl-2H-pyrazole-3-carboxylic acid [1-(3',4'-dimethyl-biphenyl-2-carbonyl)-piperidin-3-yl]-amide

LC-MS: t_{R} = 1.04 min; [M+H]⁺ = 445.53.

### Example 44: (R)-Quinoline-8-carboxylic acid [1-(3',4'-dimethyl-biphenyl-2-carbonyl)-piperidin-3-yl]-amide

LC-MS: t_{R} = 1.06 min; [M+H]⁺ = 464.59.

### Example 45: (R)-1H-Pyrrolo[2,3-b]pyridine-5-carboxylic acid [1-(3',4'-dimethyl-biphenyl-2-carbonyl)-piperidin-3-yl]-amide

LC-MS: t_{R} = 98 min; [M+H]⁺ = 453.47.

### Example 46: (R)-1H-Pyrrolo[3,2-b]pyridine-6-carboxylic acid [1-(3',4'-dimethyl-biphenyl-2-carbonyl)-piperidin-3-yl]-amide

LC-MS: t_{R} = 0.86 min; [M+H]⁺ = 453.79.

Examples 47 and 48 were prepared by applying the methods described for the preparation of Example 11 and using a 2-bromopyridine derivative as the arylating agent:

### Example 47: (R)-(3',4'-Dimethyl-biphenyl-2-yl)-13-(5-trifluoromethyl-pyridin-2-ylamino)-piperidin-1-yl]-methanone

LC-MS: t_{R} = 1.08 min; [M+H]⁺ = 454.48.

### Example 48: (R)-(3',4'-Dimethyl-biphenyl-2-yl)-[3-(5-nitro-pyridin-2-ylamino)-piperidin-1-yl]-methanone

LC-MS: t_{R} = 1.08 min; [M+H]⁺ = 431.66.

Examples 49 to 54 were prepared according to the procedures described for the preparation of Example 11 by using R-(3-amino-pyrrolidin-1-yl)-(3'-methyl-biphenyl-2-yl)-methanone as the amine in the last arylation step.

### Example 49: (R)-(3'-Methyl-biphenyl-2-yl)-[3-(5-methylsulfanyl-pyrimidin-2-ylamino)-pyrrolidin-1-yl]-methanone

LC-MS: t_{R} = 1.04 min; [M+H]⁺ = 405.55.

### Example 50: (R)-[3-(4,6-Dimethoxy-pyrimidin-2-ylamino)-pyrrolidin-1-yl]-(3'-methyl-biphenyl-2-yl)-methanone

LC-MS: t_{R} = 1.05 min; [M+H]⁺ = 419.52.

### Example 51: (R)-(3'-Methyl-biphenyl-2-yl)-[3-(quinoxalin-2-ylamino)-pyrrolidin-1-yl]-methanone

LC-MS: t_{R} = 0.99 min; [M+H]⁺ = 409.36.

### Example 52: (R)-(3'-Methyl-biphenyl-2-yl)-[3-(4-trifluoromethyl-pyrimidin-2-ylamino)-pyrrolidin-1-yl]-methanone

LC-MS: t_{R} = 1.10 min; [M+H]⁺ = 427.58.

### Example 53: (R)-[3-(Benzothiazol-2-ylamino)-pyrrolidin-1-yl]-(3'-methyl-biphenyl-2-yl)-methanone

LC-MS: t_{R} = 0.96 min; [M+H]⁺ = 414.42.

### Example 54: (R)-[3-(1H-Benzoimidazol-2-ylamino)-pyrrolidin-1-yl]-(3-methyl-biphenyl-2-yl)-methanone

LC-MS: t_{R} = 0.80 min; [M+H]⁺ = 397.41.

Examples 55 and 56 were prepared according to the procedure described for the preparation of example 15 by using R-(3-amino-pyrrolidin-1-yl)-(3'-methyl-biphenyl-2-yl)-methanone as the amine in the last acylation step.

### Example 55: (R)-1-Methyl-1H-indole-3-carboxylic acid [1-(3'-methyl-biphenyl-2-carbonyl)-pyrrolidin-3-yl]-amide

LC-MS: t_{R} = 1.00 min; [M+H]⁺ = 438.45.

### Example 56: (R)-1-Methyl-1H-indazole-3-carboxylic acid [1-(3'-methyl-biphenyl-2-carbonyl)-pyrrolidin-3-yl]-amide

LC-MS: t_{R} = 1.03 min; [M+H]⁺ = 439.09.

### II. Biological assays

### In vitro assay

The orexin receptor antagonistic activity of the compounds of formula (I) is determined in accordance with the following experimental method.

### Experimental method:

### Intracellular calcium measurements:

Chinese hamster ovary (CHO) cells expressing the human orexin-1 receptor and the human orexin-2 receptor, respectively, are grown in culture medium (Ham F-12 with L-Glutamine) containing 300 µg/ml G418, 100 U/ml penicillin, 100 µg/ml streptomycin and 10 % heat inactivated fetal calf serum (FCS). The cells are seeded at 20'000 cells / well into 384-well black clear bottom sterile plates (Greiner). The seeded plates are incubated overnight at 37°C in 5% CO₂.

Human orexin-A as an agonist is prepared as 1 mM stock solution in MeOH: water (1:1), diluted in HBSS containing 0.1 % bovine serum albumin (BSA), NaHCO₃: 0.375g/l and 20 mM HEPES for use in the assay at a final concentration of 3 nM.

Antagonists are prepared as 10 mM stock solution in DMSO, then diluted in 384-well plates using DMSO followed by a transfer of the dilutions into in HBSS containing 0.1 % bovine serum albumin (BSA), NaHCO₃: 0.375g/l and 20 mM HEPES. On the day of the assay, 50 µl of staining buffer (HBSS containing 1% FCS, 20 mM HEPES, NaHCO₃: 0.375g/l, 5 mM probenecid (Sigma) and 3 µM of the fluorescent calcium indicator fluo-4 AM (1 mM stock solution in DMSO, containing 10% pluronic) is added to each well. The 384-well cell-plates are incubated for 50 min at 37° C in 5% CO₂ followed by equilibration at RT for 30 - 120 min before measurement.

Within the Fluorescent Imaging Plate Reader (FLIPR, Molecular Devices), antagonists are added to the plate in a volume of 10 µl/well, incubated for 10 min and finally 10 µl/well of agonist is added. Fluorescence is measured for each well at 1 second intervals, and the height of each fluorescence peak is compared to the height of the fluorescence peak induced by 3 nM orexin-A with vehicle in place of antagonist. For each antagonist, the IC₅₀ value (the concentration of compound needed to inhibit 50 % of the agonistic response) is determined.

With respect to the OX₁ receptor, IC₅₀ values of 47 exemplified compounds are in the range of 6 - 8036 nM with an average of 1388 nM; IC₅₀ values of 9 compounds have been measured > 10000 nM. With respect to the OX₂ receptor, IC₅₀ values of all exemplified compounds are in the range of 8 - 4547 nM with an average of 601 nM. Antagonistic activities of selected compounds are displayed in *Table 1.*

**Table 1**

| **Compound of Example** | **OX₁ IC₅₀ (nM)** | **OX₂ IC₅₀ (nM)** |
|---|---|---|
| **1** | 58 | 43 |
| **2** | 25 | 10 |
| **6** | 13 | 11 |
| **34** | 33 | 15 |
| **41** | 32 | 25 |

## Claims

1. A compound of formula (I) 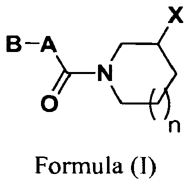 wherein
A represents a phenyl-group, wherein the phenyl is unsubstituted, or mono-substituted with (C₁₋₄)alkyl; or A represents 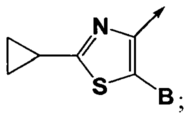 B represents phenyl, which is unsubstituted, or mono-, di-, or tri-substituted, wherein the substituents are independently selected from the group consisting of (C₁₋₄)alkyl, (C₁₋₄)alkoxy, trifluoromethyl, cyano and halogen;
n represents the integer 0 or 1;
X represents -NH-R¹ or -NH-C(O)-R²;
R¹ represents heteroaryl, wherein said heteroaryl is selected from the group consisting of pyrimidinyl, which is mono-, or di-substituted, wherein the substituents are independently selected from (C₁₋₄)alkoxy, trifluoromethyl, (C₁₋₄)alkyl-thio-, and (C₁₋₄)alkoxy-carbonyl-, or which is di-substituted, wherein one substituent is methyl and the other substituent is selected from (C₁₋₄)alkoxy, trifluoromethyl, (C₁₋₄)alkyl-thio-, and (C₁₋₄)alkoxy-carbonyl-; pyridinyl which is unsubstituted, mono-, or di-substituted, wherein the substituents are independently selected from (C₁₋₄)alkyl, (C₁₋₄)alkoxy, trifluoromethyl, halogen, (C₁₋₄)alkyl-carbonyl- and nitro; benzothiazolyl; benzimidazolyl; and quinoxalinyl which is unsubstituted, or di-substituted, wherein both substituents are independently halogen;
R² represents phenyl, which is di-substituted (notably in position 2 and 3), wherein the substituents are independently selected from (C₁₋₄)alkyl and halogen; or
R² represents heteroaryl, wherein said heteroaryl is selected from the group consisting of pyrazolyl, indolyl, indazolyl, benzisoxazolyl, quinolinyl, isoquinolinyl, imidazo[1,2-a]pyridyl, 1H-pyrrolopyridyl, and 2,3-dihydro-thieno[3,4-b][1,4]dioxinyl; wherein said groups are independently unsubstituted, mono-, or di-substituted, wherein the substituents are independently selected from (C₁₋₄)alkyl; or R² represents heterocyclyl, wherein said heterocyclyl is selected from the group consisting of 2,3-dihydro-benzo[1,4]dioxinyl, 2,3-dihydro-benzofuranyl, benzo[1,3]dioxolyl, 4*H*-benzo[1,3]dioxinyl, 2*H*-chromenyl, and chromanyl;
or a pharmaceutically acceptable salt thereof.

2. A compound of formula (I) according to claim 1, which is also a compound of formula (Ia), wherein the stereogenic center in position 3 of the piperidine or pyrrolidine ring is in absolute (R)-configuration: 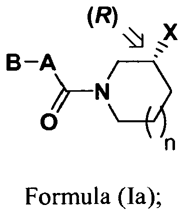 or a pharmaceutically acceptable salt thereof.

3. A compound of formula (I) according to claim 1 or 2, wherein A represents a phenyl-group, wherein the phenyl is unsubstituted, or mono-substituted with (C₁₋₄)alkyl;
or a pharmaceutically acceptable salt thereof.

4. A compound of formula (I) according to claim 1 or 2, wherein A represents 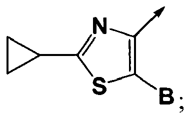 or a pharmaceutically acceptable salt thereof.

5. A compound of formula (I) according to any one of claims 1 to 4, wherein B represents phenyl, which is unsubstituted, or mono-, or di-substituted, wherein the substituents are independently selected from the group consisting of (C₁₋₄)alkyl, (C₁₋₄)alkoxy, trifluoromethyl and halogen;
or a pharmaceutically acceptable salt thereof.

6. A compound of formula (I) according to any one of claims 1 to 5, wherein X represents -NH-R¹; and
R¹ is selected from the group consisting of: 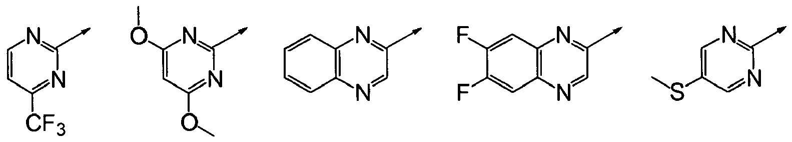 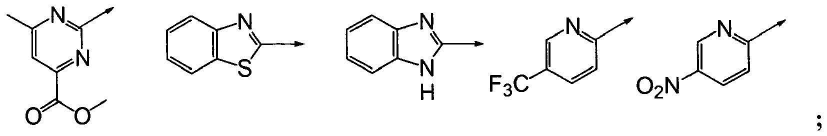 or a pharmaceutically acceptable salt thereof.

7. A compound of formula (I) according to any one of claims 1 to 5, wherein
X represents -NH-C(O)-R²; and
R² represents heteroaryl, wherein said heteroaryl is selected from the group consisting of pyrazolyl, indolyl, indazolyl, benzisoxazolyl, quinolinyl, isoquinolinyl, imidazo[1,2-a]pyridyl, 1H-pyrrolopyridyl, and 2,3-dihydro-thieno[3,4-b][1,4] dioxinyl; wherein said groups are independently unsubstituted, mono-, or di-substituted, wherein the substituents are independently selected from (C₁₋₄)alkyl; or R² represents heterocyclyl, wherein said heterocyclyl is selected from the group consisting of 2,3-dihydro-benzo[1,4]dioxinyl, 2,3-dihydro-benzofuranyl, benzo[1,3]dioxolyl, 4*H*-benzo[1,3]dioxinyl, 2*H*-chromenyl, and chromanyl;
or a pharmaceutically acceptable salt thereof.

8. A compound according to claim 1 selected from the group consisting of
(R)-(3',4'-Dimethyl-biphenyl-2-yl)-[3-(4-trifluoromethyl-pyrimidin-2-ylamino)-piperidin-1-yl]-methanone;
(R)-[3-(4,6-Dimethoxy-pyrimidin-2-ylamino)-piperidin-1-yl]-(3',4'-dimethylbiphenyl-2-yl)-methanone;
(R)-(3'-Methyl-biphenyl-2-yl)-[3-(4-trifluoromethyl-pyrimidin-2-ylamino)-piperidin-1-yl]-methanone;
(R)-[3-(4,6-Dimethoxy-pyrimidin-2-ylamino)-piperidin-1-yl]-(3'-methyl-biphenyl-2-yl)-methanone;
(R)-[3-(6,7-Difluoro-quinoxalin-2-ylamino)-piperidin-1-yl]-(3',4'-dimethyl-biphenyl-2-yl)-methanone;
(R)-(3',4'-Dimethyl-biphenyl-2-yl)-[3-(quinoxalin-2-ylamino)-piperidin-1-yl]-methanone;
(R)-(3',4'-Dimethyl-biphenyl-2-yl)-[3-(5-methylsulfanyl-pyrimidin-2-ylamino)-piperidin-1-yl]-methanone;
(R)-2-[1-(3',4'-Dimethyl-biphenyl-2-carbonyl)-piperidin-3-ylamino]-6-methylpyrimidine-4-carboxylic acid methyl ester;
(R)-[3-(Benzothiazol-2-ylamino)-piperidin-1-yl]-(3',4'-dimethyl-biphenyl-2-yl)-methanone;
(R)-[3-(1H-Benzoimidazol-2-ylamino)-piperidin-1-yl]-(3',4'-dimethyl-biphenyl-2-yl)-methanone;
(R)-[2-Cyclopropyl-5-m-tolyl-thiazol-4-yl]-[3-(4-trifluoromethyl-pyrimidin-2-ylamino)-piperidin-1-yl]-methanone;
(R)-(2-Cyclopropyl-5-m-tolyl-thiazol-4-yl)-[3-(4,6-dimethoxy-pyrimidin-2-ylamino)-piperidin-1-yl]-methanone;
(R)-[2-Cyclopropyl-5-(4-fluoro-phenyl)-thiazol-4-yl]-[3-(4-trifluoromethyl-pyrimidin-2-ylamino)-piperidin-1-yl]-methanone;
(R)-[2-Cyclopropyl-5-(4-fluoro-phenyl)-thiazol-4-yl]-[3-(4,6-dimethoxy-pyrimidin-2-ylamino)-piperidin-I-yl]-methanone; (R)-1-Methyl-1H-indazole-3-carboxylic acid [1-(2-cyclopropyl-5-m-tolyl-thiazole-4-carbonyl)-piperidin-3-yl]-amide;
(R)-2,3-Dihydro-thieno[3,4-b][1,4]dioxine-5-carboxylic acid [1-(2-cyclopropyl-5-m-tolyl-thiazole-4-carbonyl)-piperidin-3-yl]-amide;
(R)-Imidazo[1,2-a]pyridine-3-carboxylic acid [1-(2-cyclopropyl-5-m-tolyl-thiazole-4-carbonyl)-piperidin-3-yl]-amide;
(R)-Benzo[d]isoxazole-3-carboxylic acid [1-(2-cyclopropyl-5-m-tolyl-thiazole-4-carbonyl)-piperidin-3-yl]-amide;
(R)-3-Chloro-N-[1-(2-cyclopropyl-5-m-tolyl-thiazole-4-carbonyl)-piperidin-3-yl]-2-methyl-benzamide;
(R)-N-[1-(2-Cyclopropyl-5-m-tolyl-thiazole-4-carbonyl)-piperidin-3-yl]-3-fluoro-2-methyl-benzamide;
(R)-N-[1-(2-Cyclopropyl-5-m-tolyl-thiazole-4-carbonyl)-piperidin-3-yl]-2,3-dimethyl-benzamide;
(R)-2,3-Dihydro-benzo[1,4]dioxine-5-carboxylic acid [1-(2-cyclopropyl-5-m-tolyl-thiazole-4-carbonyl)-piperidin-3-yl]-amide;
(R)-1-Methyl-1H-indoie-3-carboxylic acid [1-(2-cyclopropyl-5-m-tolyl-thiazole-4-carbonyl)-piperidin-3-yl]-amide;
(R)-2-Ethyl-5-methyl-2H-pyrazole-3-carboxylic acid [1-(2-cyclopropyl-5-m-tolyl-thiazole-4-carbonyl)-piperidin-3-yl]-amide;
(R)-Quinoline-8-carboxylic acid [1-(2-cyclopropyl-5-m-tolyl-thiazole-4-carbonyl)-piperidin-3-yl]-amide;
(R)-Isoquinoline-1-carboxylic acid [1-(2-cyclopropyl-5-m-tolyl-thiazole-4-carbonyl)-piperidin-3-yl]-amide;
(R)-2,5-Dimethyl-2H-pyrazole-3-carboxylic acid [1-(2-cyclopropyl-5-m-tolyl-thiazole-4-carbonyl)-piperidin-3-yl]-amide; (R)-2,3-Dihydro-thieno[3,4-b][1,4]dioxine-5-carboxylic acid {1-[2-cyclopropyl-5-(4-fluoro-phenyl)-thiazole-4-carbonyl]-piperidin-3-yl}-amide;
(R)-Imidazo[1,2-a]pyridine-3-carboxylic acid {1-[2-cyclopropyl-5-(4-fluoro-phenyl)-thiazole-4-carbonyl]-piperidin-3-yl}-amide;
(R)-Benzo[d]isoxazole-3-carboxylic acid {1-[2-cyclopropyl-5-(4-fluoro-phenyl)-thiazole-4-carbonyl]-piperidin-3-yl}-amide;
(R)-2,3-Dihydro-benzo[1,4]dioxine-5-carboxylic acid {1-[2-cyclopropyl-5-(4-fluorophenyl)-thiazole-4-carbonyl]-piperidin-3-yl}-amide;
(R)-1-Methyl-1H-indole-3-carboxylic acid {1-[2-cyclopropyl-5-(4-fluoro-phenyl)-thiazole-4-carbonyl]-piperidin-3-yl}-amide;
(R)-Isoquinoline-1-carboxylic acid {1-[2-cyclopropyl-5-(4-fluoro-phenyl)-thiazole-4-carbonyl]-piperidin-3-yl}-amide;
(R)-1-Methyl-1H-indazole-3-carboxylic acid [1-(3'-methyl-biphenyl-2-carbonyl)-piperidin-3-yl]-amide;
(R)-1-Methyl-1H-indole-3-carboxylic acid [1-(3'-methyl-biphenyl-2-carbonyl)-piperidin-3-yl]-amide;
(R)-1H-Pyrrolo[2,3-b]pyridine-4-carboxylic acid [1-(3'-methyl-biphenyl-2-carbonyl)-piperidin-3-yl]-amide;
(R)-2-Ethyl-5-methyl-2H-pyrazole-3-carboxylic acid [1-(3'-methyl-biphenyl-2-carbonyl)-piperidin-3-yl]-amide;
(R)-Quinoline-8-carboxylic acid [1-(3'-methyl-biphenyl-2-carbonyl)-piperidin-3-yl]-amide;
(R)-1H-Pyrrolo[2,3-b]pyridine-5-carboxylic acid [1-(3'-methyl-biphenyl-2-carbonyl)-piperidin-3-yl]-amide;
(R)-1-Methyl-1H-indazole-3-carboxylic acid [1-(3',4'-dimethyl-biphenyl-2-carbonyl)-piperidin-3-yl]-amide;
(R)-1-Methyl-1H-indole-3-carboxylic acid [1-(3',4'-dimethyl-biphenyl-2-carbonyl)-piperidin-3-yl]-amide;
(R)-1H-Pyrrolo[2,3-b]pyridine-4-carboxylic acid [1-(3',4'-dimethyl-biphenyl-2-carbonyl)-piperidin-3-yl]-amide;
(R)-2-Ethyl-5-methyl-2H-pyrazole-3-carboxylic acid [1-(3',4'-dimethyl-biphenyl-2-carbonyl)-piperidin-3-yl]-amide;
(R)-Quinoline-8-carboxylic acid [1-(3',4'-dimethyl-biphenyl-2-carbonyl)-piperidin-3-yl]-amide;
(R)-1H-Pyrrolo[2,3-b]pyridine-5-carboxylic acid [1-(3',4'-dimethyl-biphenyl-2-carbonyl)-piperidin-3-yl]-amide;
(R)-1H-Pyrrolo[3,2-b]pyridine-6-carboxylic acid [1-(3',4'-dimethyl-biphenyl-2-carbonyl)-piperidin-3-yl]-amide;
(R)-(3',4'-Dimethyl-biphenyl-2-yl)-[3-(5-trifluoromethyl-pyridin-2-ylamino)-piperidin-1-yl]-methanone;
(R)-(3',4'-Dimethyl-biphenyl-2-yl)-[3-(5-nitro-pyridin-2-ylamino)-piperidin-1-yl]-methanone;
(R)-(3'-Methyl-biphenyl-2-yl)-[3-(5-methylsulfanyl-pyrimidin-2-ylamino)-pyrrolidin-1-yl]-methanone;
(R)-[3-(4,6-Dimethoxy-pyrimidin-2-ylamino)-pyrrolidin-1-yl]-(3'-methyl-biphenyl-2-yl)-methanone;
(R)-(3'-Methyl-biphenyl-2-yl)-[3-(quinoxalin-2-ylamino)-pyrrolidin-1-yl]-methanone;
(R)-(3'-Methyl-biphenyl-2-yl)-[3-(4-trifluoromethyl-pyrimidin-2-ylamino)-pyrrolidin-1-yl]-methanone;
(R)-[3-(Benzothiazol-2-ylamino)-pyrrolidin-1-yl]-(3'-methyl-biphenyl-2-yl)-methanone;
(R)-[3-(1H-Benzoimidazol-2-ylamino)-pyrrolidin-1-yl]-(3'-methyl-biphenyl-2-yl)-methanone;
(R)-1-Methyl-1H-indole-3-carboxylic acid [1-(3'-methyl-biphenyl-2-carbonyl)-pyrrolidin-3-yl]-amide; and
(R)-1-Methyl-1H-indazole-3-carboxylic acid [1-(3'-methyl-biphenyl-2-carbonyl)-pyrrolidin-3-yl]-amide;
or a pharmaceutically acceptable salt of such a compound.

9. A pharmaceutical composition containing, as active principle, a compound of formula (I) according to claim 1, or a pharmaceutically acceptable salt thereof, and at least one therapeutically inert excipient.

10. A compound of any one of claims 1 to 8, or a pharmaceutically acceptable salt thereof, for use as a medicament.

11. A compound according to any of claims 1 to 8, or a pharmaceutically acceptable salt thereof, for the prevention or treatment of a disease selected from the group consisting of all types of sleep disorders, of stress-related syndromes, of psychoactive substance use and abuse, of cognitive dysfunctions in the healthy population and in psychiatric and neurologic disorders, of eating or drinking disorders.

12. Use of a compound according to any of claims 1 to 8, or a pharmaceutically acceptable salt thereof, for the preparation of a medicament for the prevention or treatment of a disease selected from the group consisting of all types of sleep disorders, of stress-related syndromes, of psychoactive substance use and abuse, of cognitive dysfunctions in the healthy population and in psychiatric and neurologic disorders, of eating or drinking disorders.

## Patentansprüche

1. Verbindung der Formel (I) 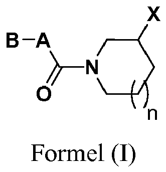 wobei
A eine Phenylgruppe repräsentiert, wobei das Phenyl unsubstituiert oder monosubstituiert ist durch (C₁₋₄)Alkyl; oder A Folgendes repräsentiert 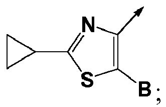 B Phenyl repräsentiert, das unsubstituiert oder mono-, di- oder trisubstituiert ist, wobei die Substituenten unabhängig ausgewählt sind aus der Gruppe bestehend aus (C₁₋₄)Alkyl, (C₁₋₄)Alkoxy, Trifluormethyl, Cyano und Halogen;
n die ganze Zahl 0 oder 1 repräsentiert;
X -NH-R¹ oder -NH-C(O)-R² repräsentiert;
R¹ Heteroaryl repräsentiert, wobei das genannte Heteroaryl ausgewählt ist aus der Gruppe bestehend aus Pyrimidinyl, das mono- oder disubstituiert ist, wobei die Substituenten unabhängig ausgewählt sind aus (C₁₋₄)Alkoxy, Trifluormethyl, (C₁₋₄)Alkyl-thio- und (C₁₋₄)Alkoxy-carbonyl-, oder das disubstituiert ist, wobei ein Substituent Methyl ist und der andere Substituent ausgewählt ist aus (C₁₋₄)Alkoxy, Trifluormethyl, (C₁₋₄)Alkyl-thio- und (C₁₋₄)Alkoxy-carbonyl-, Pyridinyl, das unsubstituiert, mono- oder disubstituiert ist, wobei die Substituenten unabhängig ausgewählt sind aus (C₁₋₄)Alkyl, (C₁₋₄)Alkoxy, Trifluormethyl, Halogen, (C₁₋₄)Alkylcarbonyl- und Nitro; Benzothiazolyl; Benzimidazolyl; und Chinoxalinyl, das unsubstituiert oder disubstituiert ist, wobei beide Substituenten unabhängig Halogen sind;
R² Phenyl repräsentiert, das disubstituiert ist (vor allem in Position 2 und 3), wobei die Substituenten unabhängig ausgewählt sind aus (C₁₋₄)Alkyl und Halogen; oder
R² Heteroaryl repräsentiert, wobei das genannte Heteroaryl ausgewählt ist aus der Gruppe bestehend aus Pyrazolyl, Indolyl, Indazolyl, Benzisoxazolyl, Chinolinyl, Isochinolinyl, Imidazo[1,2-a]pyridyl, 1H-Pyrrolopyridyl und 2,3-Dihydro-thieno[3,4-b][1,4]dioxinyl; wobei die genannten Gruppen unabhängig unsubstituiert, mono- oder disubstituiert sind, wobei die Substituenten unabhängig ausgewählt sind aus (C₁₋₄)Alkyl; oder
R² Heterocyclyl repräsentiert, wobei das genannte Heterocyclyl ausgewählt ist aus der Gruppe bestehend aus 2,3-Dihydro-benzo[1,4]dioxinyl, 2,3-Dihydro-benzofuranyl, Benzo[1,3]dioxolyl, 4*H*-Benzo[1,3]dioxinyl, 2*H*-Chromenyl und Chromanyl;
oder ein pharmazeutisch akzeptables Salz davon.

2. Verbindung der Formel (I) nach Anspruch 1, die auch eine Verbindung der Formel (Ia) ist, wobei das stereogene Zentrum in Position 3 des Piperidin- oder Pyrrolidinrings in absoluter (R)-Konfiguration ist: 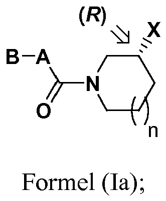 oder ein pharmazeutisch akzeptables Salz davon.

3. Verbindung der Formel (I) nach Anspruch 1 oder 2, wobei A eine Phenyl-Gruppe repräsentiert, wobei das Phenyl unsubstituiert oder monosubstituiert ist durch (C₁₋₄)Alkyl;
oder ein pharmazeutisch akzeptables Salz davon.

4. Verbindung der Formel (I) nach Anspruch 1 oder 2, wobei A Folgendes repräsentiert 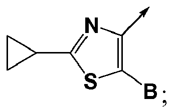 oder ein pharmazeutisch akzeptables Salz davon.

5. Verbindung der Formel (I) nach einem der Ansprüche 1 bis 4, wobei B Phenyl repräsentiert, das unsubstituiert oder mono- oder disubstituiert ist, wobei die Substituenten unabhängig ausgewählt sind aus der Gruppe bestehend aus (C₁₋₄)Alkyl, (C₁₋₄)Alkoxy, Trifluormethyl und Halogen;
oder ein pharmazeutisch akzeptables Salz davon.

6. Verbindung der Formel (I) nach einem der Ansprüche 1 bis 5, wobei X -NH-R¹ repräsentiert; und
R¹ ausgewählt ist aus der Gruppe bestehend aus: 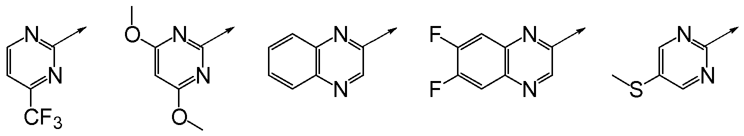 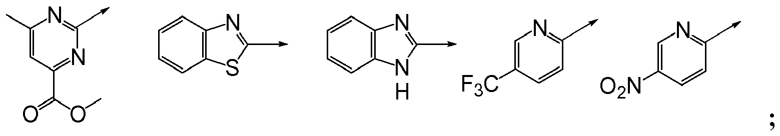 oder ein pharmazeutisch akzeptables Salz davon.

7. Verbindung der Formel (I) nach einem der Ansprüche 1 bis 5, wobei X -NH-C(O)-R² repräsentiert; und
R² Heteroaryl repräsentiert, wobei das genannte Heteroaryl ausgewählt ist aus der Gruppe bestehend aus Pyrazolyl, Indolyl, Indazolyl, Benzisoxazolyl, Chinolinyl, Isochinolinyl, Imidazo[1,2-a]pyridyl, 1H-Pyrrolopyridyl und 2,3-Dihydro-thieno[3,4-b][1,4] dioxinyl; wobei die genannten Gruppen unabhängig unsubstituiert, mono- oder disubstituiert sind, wobei die Substituenten unabhängig ausgewählt sind aus (C₁₋₄)Alkyl; oder
R² Heterocyclyl repräsentiert, wobei das genannte Heterocyclyl ausgewählt ist aus der Gruppe bestehend aus 2,3-Dihydro-benzo[1,4]dioxinyl, 2,3-Dihydro-benzofuranyl, Benzo[1,3]dioxolyl, 4*H*-Benzo[1,3]dioxinyl, 2*H*-Chromenyl und Chromanyl;
oder ein pharmazeutisch akzeptables Salz davon.

8. Verbindung nach Anspruch 1, die ausgewählt ist aus der Gruppe bestehend aus
(R)-(3',4'-Dimethyl-biphenyl-2-yl)-[3-(4-trifluormethyl-pyrimidin-2-ylamino)-piperidin-1-yl] -methanon;
(R)-[3-(4,6-Dimethoxy-pyrimidin-2-ylamino)-piperidin-1-yl]-(3',4'-dimethyl-biphenyl-2-yl)-methanon;
(R)-(3'-Methyl-biphenyl-2-yl)-[3-(4-tri fluormethyl-pyrimidin-2-ylamino)-piperidin-1-yl]-methanon;
(R)-[3-(4,6-Dimethoxy-pyrimidin-2-ylamino)-piperidin-1-yl]-(3'-methyl-biphenyl-2-yl)-methanon;
(R)-[3-(6,7-Difluor-chinoxalin-2-ylamino)-piperidin-1-yl]-(3',4'-dimethyl-biphenyl-2-yl)-methanon;
(R)-(3',4'-Dimethyl-biphenyl-2-yl)-[3-(chinoxalin-2-ylamino)-piperidin-1-yl]-methanon;
(R)-(3',4'-Dimethyl-biphenyl-2-yl)-[3-(5-methylsulfanyl-pyrimidin-2-ylamino)-piperidin-1-yl]-methanon;
(R)-2-[1-(3',4'-Dimethyl-biphenyl-2-carbonyl)-piperidin-3-ylamino]-6-methyl-pyrimidin-4-carbonsäuremethylester;
(R)-[3-(Benzothiazol-2-ylamino)-piperidin-1-yl]-(3',4'-dimethyl-biphenyl-2-yl)-methanon;
(R)-[3-(1H-Benzoimidazol-2-ylamino)-piperidin-1-yl]-(3',4'-dimethyl-biphenyl-2-yl)-methanon;
(R)-[2-Cyclopropyl-5-m-tolyl-thiazol-4-yl]-[3-(4-trifluormethyl-pyrimidin-2-ylamino)-piperidin-1-yl]-methanon,
(R)-(2-Cyclopropyl-5-m-tolyl-thiazol-4-yl)-[3-(4,6-dimethoxy-pyrimidin-2-ylamino)-piperidin-1-yl]-methanon;
(R)-[2-Cyclopropyl-5-(4-fluor-phenyl)-thiazol-4-yl]-[3-(4-trifluormethyl-pyrimidin-2-ylamino)-piperidin-1-yl]-methanon;
(R)-[2-Cyclopropyl-5-(4-fluor-phenyl)-thiazol-4-yl]-[3-(4,6-dimethoxy-pyrimidin-2-ylamino)-piperidin-1-yl]-methanon;
(R)-1-Methyl-1H-indazol-3-carbonsäure [1-(2-cyclopropyl-5-m-tolyl-thiazol-4-carbonyl)-piperidin-3-yl]-amid;
(R)-2,3-Dihydro-thieno[3,4-b][1,4]dioxin-5-carbonsäure [1-(2-cyclopropyl-5-m-tolyl-thiazol-4-carbonyl)-piperidin-3-yl]-amid;
(R)-Imidazo[1,2-a]pyridin-3-carbonsäure [1-(2-cyclopropyl-5-m-tolyl-thiazol-4-carbonyl)-piperidin-3-yl]-amid;
(R)-Benzo[d]isoxazol-3-carbonsäure [1-(2-cyclopropyl-5-m-tolyl-thiazol-4-carbonyl)-piperidin-3-yl]-amid;
(R)-3-Chlor-N-[1-(2-cyclopropyl-5-m-tolyl-thiazol-4-carbonyl)-piperidin-3-yl]-2-methyl-benzamid;
(R)-N-[1-(2-Cyclopropyl-5-m-tolyl-thiazol-4-carbonyl)-piperidin-3-yl]-3-fluor-2-methyl-benzamid;
(R)-N-[1-(2-Cyclopropyl-5-m-tolyl-thiazol-4-carbonyl)-piperidin-3-yl]-2,3-dimethyl-benzamid;
(R)-2,3-Dihydro-benzo[1,4]dioxin-5-carbonsäure [1-(2-cyclopropyl-5-m-tolyl-thiazol-4-carbonyl)-piperidin-3-yl]-amid;
(R)-1-Methyl-1H-indol-3-carbonsäure [1-(2-cyclopropyl-5-m-tolyl-thiazol-4-carbonyl)-piperidin-3-yl]-amid;
(R)-2-Ethyl-5-methyl-2H-pyrazol-3-carbonsäure [1-(2-cyclopropyl-5-m-tolyl-thiazol-4-carbonyl)-piperidin-3-yl]-amid;
(R)-Chinolin-8-carbonsäure [1-(2-cyclopropyl-5-m-tolyl-thiazol-4-carbonyl)-piperidin-3-yl]-amid;
(R)-Isochinolin-1-carbonsäure [1-(2-cyclopropyl-5-m-tolyl-thiazol-4-carbonyl)-piperidin-3-yl]-amid;
(R)-2,5-Dimethyl-2H-pyrazol-3-carbonsäure [1-(2-cyclopropyl-5-m-tolyl-thiazol-4-carbonyl)-piperidin-3-yl]-amid;
(R)-2,3-Dihydro-thieno[3,4-b][1,4]dioxin-5-carbonsäure {1-[2-cyclopropyl-5-(4-fluor-phenyl)-thiazol-4-carbonyl]-piperidin-3-yl}-amid;
(R)-Imidazo[1,2-a]pyridin-3-carbonsäure {1-[2-cyclopropyl-5-(4-fluor-phenyl)-thiazol-4-carbonyl]-piperidin-3-yl}-amid;
(R)-Benzo[d]isoxazol-3-carbonsäure {1-[2-cyclopropyl-5-(4-fluor-phenyl)-thiazol-4-carbonyl]-piperidin-3-yl}-amid;
(R)-2,3-Dihydro-benzo[1,4]dioxin-5-carbonsäure {1-[2-cyclopropyl-5-(4-fluorphenyl)-thiazol-4-carbonyl]-piperidin-3-yl}-amid;
(R)-1-Methyl-1H-indol-3-carbonsäure {1-[2-cyclopropyl-5-(4-fluor-phenyl)-thiazol-4-carbonyl]-piperidin-3-yl}-amid;
(R)-Isochinolin-1-carbonsäure {1-[2-cyclopropyl-5-(4-fluor-phenyl)-thiazol-4-carbonyl]-piperidin-3-yl} -amid;
(R)-1-Methyl-1H-indazol-3-carbonsäure [1-(3'-methyl-biphenyl-2-carbonyl)-piperidin-3-yl]-amid;
(R)-1-Methyl-1H-indol-3-carbonsäure [1-(3'-methyl-biphenyl-2-carbonyl)-piperidin-3-yl]-amid;
(R)-1H-Pyrrolo[2,3-b]pyridin-4-carbonsäure [1-(3'-methyl-biphenyl-2-carbonyl)-piperidin-3-yl]-amid;
(R)-2-Ethyl-5-methyl-2H-pyrazol-3-carbonsäure [1-(3'-methyl-biphenyl-2-carbonyl)-piperidin-3-yl]-amid;
(R)-Chinolin-8-carbonsäure [1-(3'-methyl-biphenyl-2-carbonyl)-piperidin-3-yl]-amid;
(R)-1H-Pyrrolo[2,3-b]pyridin-5-carbonsäure [1-(3'-methyl-biphenyl-2-carbonyl)-piperidin-3-yl]-amid;
(R)-1-Methyl-1H-indazol-3-carbonsäure [1-(3',4'-dimethyl-biphenyl-2-carbonyl)-piperidin-3-yl]-amid;
(R)-1-Methyl-1H-indol-3-carbonsäure [1-(3',4'-dimethyl-biphenyl-2-carbonyl)-piperidin-3-yl]-amid;
(R)-1H-Pyrrolo[2,3-b]pyridin-4-carbonsäure [1-(3',4'-dimethyl-biphenyl-2-carbonyl)-piperidin-3-yl]-amid;
(R)-2-Ethyl-5-methyl-2H-pyrazol-3-carbonsäure [1-(3',4'-dimethyl-biphenyl-2-carbonyl)-piperidin-3-yl]-amid;
(R)-Chinolin-8-carbonsäure [1-(3',4'-dimethyl-biphenyl-2-carbonyl)-piperidin-3-yl]-amid;
(R)-1H-Pyrrolo[2,3-b]pyridin-5-carbonsäure [1-(3',4'-dimethyl-biphenyl-2-carbonyl)-piperidin-3-yl]-amid;
(R)-1H-Pyrrolo[3,2-b]pyridin-6-carbonsäure [1-(3',4'-dimethyl-biphenyl-2-carbonyl)-piperidin-3-yl]-amid;
(R)-(3',4'-Dimethyl-biphenyl-2-yl)-[3-(5-trifluormethyl-pyridin-2-ylamino)-piperidin-1-yl]-methanon;
(R)-(3',4'-Dimethyl-biphenyl-2-yl)-[3-(5-nitro-pyridin-2-ylamino)-piperidin-1-yl]-methanon;
(R)-(3'-Methyl-biphenyl-2-yl)-[3-(5-methylsulfanyl-pyrimidin-2-ylamino)-pyrrolidin-1-yl]-methanon;
(R)-[3-(4,6-Dimethoxy-pyrimidin-2-ylamino)-pyrrolidin-1-yl]-(3'-methyl-biphenyl-2-yl)-methanon;
(R)-(3'-Methyl-biphenyl-2-yl)-[3-(chinoxalin-2-ylamino)-pyrrolidin-1-yl]-methanon;
(R)-(3'-Methyl-biphenyl-2-yl)-[3-(4-trifluormethyl-pyrimidin-2-ylamino)-pyrrolidin-1-yl]-methanon;
(R)-[3-(Benzothiazol-2-ylamino)-pyrrolidin-1-yl]-(3'-methyl-biphenyl-2-yl)-methanon;
(R)-[3-(1H-Benzoimidazol-2-ylamino)-pyrrolidin-1-yl]-(3'-methyl-biphenyl-2-yl)-methanon;
(R)-1-Methyl-1H-indol-3-carbonsäure [1-(3'-methyl-biphenyl-2-carbonyl)-pyrrolidin-3-yl]-amid; und
(R)-1-Methyl-1H-indazol-3-carbonsäure [1-(3'-methyl-biphenyl-2-carbonyl)-pyrrolidin-3-yl]-amid;
oder ein pharmazeutisch akzeptables Salz einer solchen Verbindung.

9. Pharmazeutische Zusammensetzung, die als Wirkstoff eine Verbindung der Formel (I) nach Anspruch 1 oder ein pharmazeutisch akzeptables Salz davon und wenigstens einen therapeutisch inerten Exzipienten enthält.

10. Verbindung nach einem der Ansprüche 1 bis 8 oder ein pharmazeutisch akzeptables Salz davon zur Verwendung als Medikament.

11. Verbindung nach einem der Ansprüche 1 bis 8 oder ein pharmazeutisch akzeptables Salz davon zur Verhütung oder Behandlung einer Krankheit, die ausgewählt ist aus der Gruppe bestehend aus allen Arten von Schlafstörungen, stressbedingten Syndromen, Psychotropikumgebrauch und -missbrauch, kognitiven Fehlfunktionen bei der gesunden Bevölkerung und bei psychiatrischen und neurologischen Störungen, Ess- oder Trinkstörungen.

12. Verwendung einer Verbindung nach einem der Ansprüche 1 bis 8 oder eines pharmazeutisch akzeptablen Salzes davon zur Herstellung eines Medikaments zur Verhütung oder Behandlung einer Krankheit, die ausgewählt ist aus der Gruppe bestehend aus allen Arten von Schlafstörungen, stressbedingten Syndromen, Psychotropikumgebrauch und -missbrauch, kognitiven Fehlfunktionen bei der gesunden Bevölkerung und bei psychiatrischen und neurologischen Störungen, Ess - oder Trinkstörungen.

## Revendications

1. Composé de formule (I) 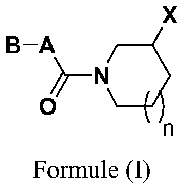 où
A représente un groupement phényle, où le phényle est non substitué ou monosubstitué par un (C₁₋₄)alkyle ; ou A représente 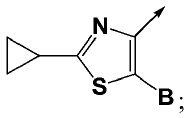 B représente un phényle qui est non substitué ou mono-, di- ou trisubstitué par des substituants indépendamment sélectionnés dans le groupe consistant en un (C₁₋₄)alkyle, (C₁₋₄)alkoxy, trifluorométhyle, cyano et halogène ;
n représente le nombre entier 0 ou 1 ;
X représente -NH-R¹ ou -NH-C(O)-R² ;
R¹ représente un hétéroaryle, où ledit hétéroaryle est sélectionné dans le groupe consistant en un pyrimidinyle qui est mono- ou disubstitué par des substituants indépendamment sélectionnés parmi un (C₁₋₄)alkoxy, trifluorométhyle, (C₁₋₄)alkyl-thio- et (C₁₋₄)alkoxy-carbonyle ou qui est disubstitué par un méthyle et un autre substituant sélectionné parmi un (C₁₋₄)alkoxy, trifluorométhyle, (C₁₋₄)alkyl-thio- et (C₁₋₄)alkoxy-carbonyle ; un pyridinyle qui est non substitué ou mono- ou disubstitué par des substituants indépendamment sélectionnés parmi un (C₁₋₄)alkyle, (C₁₋₄)alkoxy, trifluorométhyle, halogène, (C₁₋₄)alkyl-carbonyle et nitro ; un benzothiazolyle ; un benzimidazolyle ; et un quinoxalinyle qui est non substitué ou disubstitué par des substituants tous deux indépendamment sélectionnés parmi des halogènes ;
R² représente un phényle qui est disubstitué (notamment en positions 2 et 3) par des substituants indépendamment sélectionnés parmi un (C₁₋₄)alkyle et un halogène ; ou
R² représente un hétéroaryle, où ledit hétéroaryle est sélectionné dans le groupe consistant en un pyrazolyle, indolyle, indazolyle, benzisoxazolyle, quinolinéyle, isoquinolinéyle, imidazo[1,2-a]pyridyle, 1*H*-pyrrolopyridyle et 2,3-dihydro-thiéno[3,4-b][1,4]dioxinyle ; où lesdits groupements sont indépendamment non substitués ou mono- ou disubstitués par des substituants indépendamment sélectionnés parmi des (C₁₋₄)alkyles ; ou
R² représente un hétérocyclyle, où ledit hétérocyclyle est sélectionné dans le groupe consistant en un 2,3-dihydro-benzo[1,4]dioxinyle, 2,3-dihydro-benzofuranyle, benzo[1,3]dioxolyle, 4*H*-benzo[1,3]dioxinyle, 2*H*-chroményle et chromanyle ;
ou sel pharmaceutiquement acceptable de celui-ci.

2. Composé de formule (I) selon la revendication 1 qui est également un composé de formule (Ia), où le centre stéréogène en position 3 du noyau pipéridine ou pyrrolidine est en configuration absolue (R) : 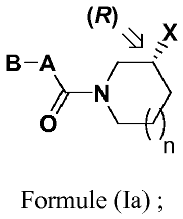 ou sel pharmaceutiquement acceptable de celui-ci.

3. Composé de formule (I) selon la revendication 1 ou 2 où A représente un groupement phényle, où le phényle est non substitué ou monosubstitué par un (C₁₋₄)alkyle ;
ou sel pharmaceutiquement acceptable de celui-ci.

4. Composé de formule (I) selon la revendication 1 ou 2, où A représente 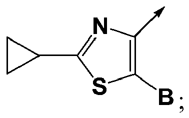 ou sel pharmaceutiquement acceptable de celui-ci.

5. Composé de formule (I) selon l'une quelconque des revendications 1 à 4, où B représente un phényle qui est non substitué ou mono- ou disubstitué par des substituants indépendamment sélectionnés dans le groupe consistant en un (C₁₋₄)alkyle, (C₁₋₄)alkoxy, trifluorométhyle et halogène ;
ou sel pharmaceutiquement acceptable de celui-ci.

6. Composé de formule (I) selon l'une quelconque des revendications 1 à 5, où X représente -NH-R¹ ; et
R¹ est sélectionné dans le groupe consistant en : 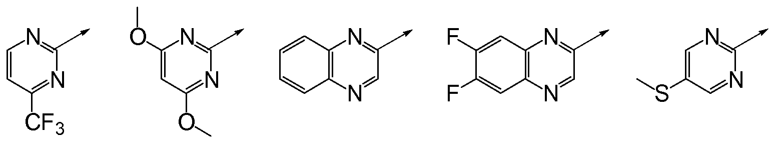 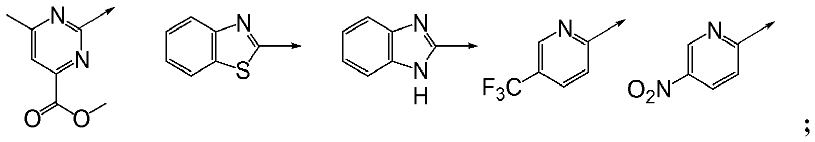 ou sel pharmaceutiquement acceptable de celui-ci.

7. Composé de formule (I) selon l'une quelconque des revendications 1 à 5, où X représente -NH-C(O)-R² ; et
R² représente un hétéroaryle, où ledit hétéroaryle est sélectionné dans le groupe consistant en un pyrazolyle, indolyle, indazolyle, benzisoxazolyle, quinolinéyle, isoquinolinéyle, imidazo[1,2-a]pyridyle, 1*H*-pyrrolopyridyle et 2,3-dihydrothiéno[3,4-b][1,4]dioxinyle ; où lesdits groupements sont indépendamment non substitués ou mono- ou disubstitués par des substituants indépendamment sélectionnés parmi des (C₁₋₄)alkyles ; ou
R² représente un hétérocyclyle, où ledit hétérocyclyle est sélectionné dans le groupe consistant en un 2,3-dihydro-benzo[1,4]dioxinyle, 2,3-dihydro-benzofuranyle, benzo[1,3]dioxolyle, 4*H*-benzo[1,3]dioxinyle, 2H-chroményle et chromanyle ;
ou sel pharmaceutiquement acceptable de celui-ci.

8. Composé selon la revendication 1, sélectionné dans le groupe consistant en les suivants :
(R)-(3',4'-Diméthyl-biphényl-2-yl)-[3-(4-trifluorométhyl-pyrimidin-2-ylamino)-pipéridin-1-yl]-méthanone ;
(R)-[3-(4,6-Diméthoxy-pyrimidin-2-ylamino)-pipéridin-1-yl]-(3',4'-diméthyl-biphényl-2-yl)-méthanone ;
(R)-(3'-Méthyl-biphényl-2-yl)-[3-(4-trifluorométhyl-pyrimidin-2-ylamino)-pipéridin-1-yl]-méthanone ;
(R)-[3-(4,6-Diméthoxy-pyrimidin-2-ylamino)-pipéridin-1-yl]-(3'-méthyl-biphényl-2-yl)-méthanone ;
(R)-[3-(6,7-Difluoro-quinoxalin-2-ylamino)-pipéridin-1-yl]-(3',4'-diméthyl-biphényl-2-yl)-méthanone ;
(R)-(3',4'-Diméthyl-biphényl-2-yl)-[3-(quinoxalin-2-ylamino)-pipéridin-1-yl]-méthanone ;
(R)-(3',4'-Diméthyl-biphényl-2-yl)-[3-(5-méthylsulfanyl-pyrimidin-2-ylamino)-pipéridin-1-yl]-méthanone ; Ester méthylique de l'acide (R)-2-[1-(3',4'-diméthyl-biphényl-2-carbonyl)-pipéridin-3-ylamino]-6-méthyl-pyrimidine-4-carboxylique ;
(R)-[3-(Benzothiazol-2-ylamino)-pipéridin-1-yl]-(3',4'-diméthyl-biphényl-2-yl)-méthanone ;
(R)-[3-(1*H*-Benzoimidazol-2-ylamino)-pipéridin-1-yl]-(3',4'-diméthyl-biphényl-2-yl)-méthanone ;
(R)-[2-Cyclopropyl-5-*m*-tolyl-thiazol-4-yl]-[3-(4-trifluorométhyl-pyrimidin-2-ylamino)-pipéridin-1-yl]-méthanone ;
(R)-(2-Cyclopropyl-5-*m*-tolyl-thiazol-4-yl)-[3-(4,6-diméthoxy-pyrimidin-2-ylamino)-pipéridin-1-yl]-méthanone ;
(R)-[2-Cyclopropyl-5-(4-fluoro-phényl)-thiazol-4-yl]-[3-(4-trifluorométhyl-pyrimidin-2-ylamino)-pipéridin-1-yl]-méthanone ;
(R)-[2-Cyclopropyl-5-(4-fluoro-phényl)-thiazol-4-yl]-[3-(4,6-diméthoxy-pyrimidin-2-ylamino)-pipéridin-1-yl]-méthanone ;
[1-(2-Cyclopropyl-5-*m*-tolyl-thiazol-4-carbonyl)-pipéridin-3-yl]-amide de l'acide (R)-1-méthyl-1*H*-indazole-3-carboxylique ;
[1-(2-Cyclopropyl-5-*m*-tolyl-thiazol-4-carbonyl)-pipéridin-3-yl]-amide de l'acide (R)-2,3-dihydro-thiéno[3,4-b][1,4]dioxine-5-carboxylique ;
[1-(2-Cyclopropyl-5-*m*-tolyl-thiazol-4-carbonyl)-pipéridin-3-yl]-amide de l'acide (R)-imidazo[1,2-a]pyridine-3-carboxylique ; [1-(2-Cyclopropyl-5-*m*-tolyl-thiazol-4-carbonyl)-pipéridin-3-yl]-amide de l'acide (R)-benzo[d]isoxazole-3-carboxylique ;
(R)-3-Chloro-N-[1-(2-cyclopropyl-5-*m*-tolyl-thiazole-4-carbonyl)-pipéridin-3-yl]-2-méthyl-benzamide ;
(R)-N-[1-(2-Cyclopropyl-5-*m*-tolyl-thiazol-4-carbonyl)-pipéridin-3-yl]-3-fluoro-2-méthyl-benzamide ;
(R)-N-[1-(2-Cyclopropyl-5-*m*-tolyl-thiazol-4-carbonyl)-pipéridin-3-yl]-2,3-diméthyl-benzamide ;
[1-(2-Cyclopropyl-5-*m*-tolyl-thiazol-4-carbonyl)-pipéridin-3-yl]-amide de l'acide (R)-2,3-dihydro-benzo[1,4]dioxine-5-carboxylique ;
[1-(2-Cyclopropyl-5-*m*-tolyl-thiazol-4-carbonyl)-pipéridin-3-yl]-amide de l'acide (R)-1-méthyl-1*H*-indole-3-carboxylique ;
[1-(2-Cyclopropyl-5-*m*-tolyl-thiazol-4-carbonyl)-pipéridin-3-yl]-amide de l'acide (R)-2-éthyl-5-méthyl-2*H*-pyrazole-3-carboxylique;
[1-(2-Cyclopropyl-5-*m*-tolyl-thiazol-4-carbonyl)-pipéridin-3-yl]-amide de l'acide (R)-quinoléine-8-carboxylique ;
[1-(2-Cyclopropyl-5-*m*-tolyl-thiazol-4-carbonyl)-pipéridin-3-yl]-amide de l'acide (R)-isoquinoléine-1-carboxylique ;
[1-(2-Cyclopropyl-5-*m*-tolyl-thiazol-4-carbonyl)-pipéridin-3-yl]-amide de l'acide (R)-2,5-diméthyl-2*H*-pyrazole-3-carboxylique;
{1-[2-Cyclopropyl-5-(4-fluoro-phényl)-thiazol-4-carbonyl]-pipéridin-3-yl}-amide de l'acide (R)-2,3-dihydro-thiéno[3,4-b][1,4]dioxine-5-carboxylique ;
{1-[2-Cyclopropyl-5-(4-fluoro-phényl)-thiazol-4-carbonyl]-pipéridin-3-yl}-amide de l'acide (R)-imidazo[1,2-a]pyridine-3-carboxylique ;
{1-[2-Cyclopropyl-5-(4-fluoro-phényl)-thiazol-4-carbonyl]-pipéridin-3-yl}-amide de l'acide (R)-benzo[d]isoxazole-3-carboxylique ;
{1-[2-Cyclopropyl-5-(4-fluoro-phényl)-thiazol-4-carbonyl]-pipéridin-3-yl}-amide de l'acide (R)-2,3-dihydro-benzo[1,4]dioxine-5-carboxylique ;
{1-[2-Cyclopropyl-5-(4-fluoro-phényl)-thiazol-4-carbonyl]-pipéridin-3-yl}-amide de l'acide (R)-1-méthyl-1*H*-indole-3-carboxylique ;
{1-[2-Cyclopropyl-5-(4-fluoro-phényl)-thiazol-4-carbonyl]-pipéridin-3-yl}-amide de l'acide (R)-isoquinoléine-1-carboxylique ;
[1-(3'-Méthyl-biphényl-2-carbonyl)-pipéridin-3-yl]-amide de l'acide (R)-1-méthyl-1*H*-indazole-3-carboxylique ;
[1-(3'-Méthyl-biphényl-2-carbonyl)-pipéridin-3-yl]-amide de l'acide (R)-1-méthyl-1*H*-indole-3-carboxylique ;
[1-(3'-Méthyl-biphényl-2-carbonyl)-pipéridin-3-yl]-amide de l'acide (R)-1*H-*pyrrolo[2,3-b]pyridine-4-carboxylique ;
[1-(3'-Méthyl-biphényl-2-carbonyl)-pipéridin-3-yl]-amide de l'acide (R)-2-éthyl-5-méthyl-2*H*-pyrazole-3-carboxylique ;
[1-(3'-Méthyl-biphényl-2-carbonyl)-pipéridin-3-yl]-amide de l'acide (R)-quinoléine-8-carboxylique ;
[1-(3'-Méthyl-biphényl-2-carbonyl)-pipéridin-3-yl]-amide de l'acide (R)-1*H-*pyrrolo[2,3-b]pyridine-5-carboxylique ;
[1-(3',4'-Diméthyl-biphényl-2-carbonyl)-pipéridin-3-yl]-amide de l'acide (R)-1-méthyl-1*H*-indazole-3-carboxylique ;
[1-(3',4'-Diméthyl-biphényl-2-carbonyl)-pipéridin-3-yl]-amide de l'acide (R)-1-méthyl-1*H*-indole-3-carboxylique ;
[1-(3',4'-Diméthyl-biphényl-2-carbonyl)-pipéridin-3-yl]-amide de l'acide (R)-1*H-*pyrrolo[2,3-b]pyridine-4-carboxylique ;
[1-(3',4'-Diméthyl-biphényl-2-carbonyl)-pipéridin-3-yl]-amide de l'acide (R)-2-éthyl-5-méthyl-2*H*-pyrazole-3-carboxylique ;
[1-(3',4'-Diméthyl-biphényl-2-carbonyl)-pipéridin-3-yl]-amide de l'acide (R)-quinoléine-8-carboxylique ;
[1-(3',4'-Diméthyl-biphényl-2-carbonyl)-pipéridin-3-yl]-amide de l'acide (R)-1*H-*pyrrolo[2,3-b]pyridine-5-carboxylique ;
[1-(3',4'-Diméthyl-biphényl-2-carbonyl)-pipéridin-3-yl]-amide de l'acide (R)-1*H-*pyrrolo[3,2-b]pyridine-6-carboxylique ;
(R)-(3',4'-Diméthyl-biphényl-2-yl)-[3-(5-trifluorométhyl-pyridin-2-ylamino)-pipéridin-1-yl]-méthanone ;
(R)-(3',4'-Diméthyl-biphényl-2-yl)-[3-(5-nitro-pyridin-2-ylamino)-pipéridin-1-yl]-méthanone ;
(R)-(3'-Méthyl-biphényl-2-yl)-[3-(5-méthylsulfanyl-pyrimidin-2-ylamino)-pyrrolidin-1-yl]-méthanone ;
(R)-[3-(4,6-Diméthoxy-pyrimidin-2-ylamino)-pyrrolidin-1-yl]-(3'-méthyl-biphényl-2-yl)-méthanone ;
(R)-(3'-Méthyl-biphényl-2-yl)-[3-(quinoxalin-2-ylamino)-pyrrolidin-1-yl]-méthanone ;
(R)-(3'-Méthyl-biphényl-2-yl)-[3-(4-trifluorométhyl-pyrimidin-2-ylamino)-pyrrolidin-1-yl]-méthanone ; (R)-[3-(Benzothiazol-2-ylamino)-pyrrolidin-1-yl]-(3'-méthyl-biphényl-2-yl)-méthanone ;
(R)- [3-(1H-Benzoimidazol-2-ylamino)-pyrrolidin-1-yl]-(3'-méthyl-biphényl-2-yl)-méthanone ;
[1-(3'-Méthyl-biphényl-2-carbonyl)-pyrrolidin-3-yl]-amide de l'acide (R)-1-méthyl-1*H*-indole-3-carboxylique; et
[1-(3'-Méthyl-biphényl-2-carbonyl)-pyrrolidin-3-yl]-amide de l'acide (R)-1-méthyl-1*H*-indazole-3-carboxylique ;
ou sel pharmaceutiquement acceptable de celui-ci.

9. Composition pharmaceutique contenant, comme principe actif, un composé de formule (I) selon la revendication 1 ou un sel pharmaceutiquement acceptable de celui-ci et au moins un excipient inerte sur le plan thérapeutique.

10. Composé de l'une quelconque des revendications 1 à 8 ou sel pharmaceutiquement acceptable de celui-ci, en vue d'une utilisation comme médicament.

11. Composé selon l'une quelconque des revendications 1 à 8 ou sel pharmaceutiquement acceptable de celui-ci, pour la prévention ou le traitement d'une maladie sélectionnée dans le groupe consistant en tous les types de troubles du sommeil, de syndromes liés à un stress, d'usage ou d'abus de substances psychoactives, de dysfonctionnements cognitifs dans la population saine et chez des patients atteints de maladies psychiatriques et neurologiques et de troubles du comportement alimentaire ou perturbations des apports de liquides.

12. Utilisation d'un composé selon l'une quelconque des revendications 1 à 8 ou d'un sel pharmaceutiquement acceptable de celui-ci dans la préparation d'un médicament indiqué dans la prévention ou le traitement d'une maladie sélectionnée dans le groupe consistant en tous les types de troubles du sommeil, de syndromes liés à un stress, d'usage ou d'abus de substances psychoactives, de dysfonctionnements cognitifs dans la population saine et chez des patients atteints de maladies psychiatriques et neurologiques et de troubles du comportement alimentaire ou perturbations des apports de liquides.
